(19) Europäisches Patentamt
European Patent Office
Office européen des brevets

(11) EP 0 609 960 B1

(12) **EUROPEAN PATENT SPECIFICATION**

(45) Date of publication and mention of the grant of the patent:
**03.03.1999 Bulletin 1999/09**

(51) Int. Cl.$^6$: **C07D 213/75**, A61K 31/44, C07D 239/52, C07D 239/58, A61K 31/505

(21) Application number: **94200437.5**

(22) Date of filing: **13.09.1990**

(54) **New N-heteroarylamide derivatives as inhibitors of acyl coenzyme A: cholestrol acyl transferase**

N-Heteroarylamidederivate als Inhibitoren von Acyl-Coenzym A: Cholesterol-Acyl-Transférase

Dérivés de N-hétéroarylamide comme inhibiteurs d'acyl coenzym A: cholestérol-acyl-transferase

(84) Designated Contracting States:
**AT BE CH DE DK ES FR GB GR IT LI LU NL SE**

(30) Priority: **15.09.1989 US 4033**

(43) Date of publication of application:
**10.08.1994 Bulletin 1994/32**

(62) Document number(s) of the earlier application(s) in accordance with Art. 76 EPC:
**90310009.7 / 0 418 071**

(73) Proprietor: **PFIZER INC.**
**New York, N.Y. 10017 (US)**

(72) Inventors:
- **McCarthy, Peter A.**
  **RR5, Pawcatuck, Connecticut (US)**
- **Hamanaka, Ernest S.**
  **Gales Ferry, Connecticut (US)**
- **Walker, Frederick J.**
  **Preston, Connecticut (US)**
- **Chang, George**
  **Ivoryton, Connecticut 06442 (US)**
- **Truong, Thien**
  **Old Saybrook, Connecticut 06475 (US)**

(74) Representative:
**Moore, James William, Dr.**
**Pfizer Limited**
**Ramsgate Road**
**Sandwich Kent CT13 9NJ (GB)**

(56) References cited:

| | |
|---|---|
| **EP-A- 0 245 687** | **EP-A- 0 283 742** |
| **WO-A-91/04027** | **FR-M- 4 801** |
| **US-A- 3 555 035** | |

- **CHEMICAL ABSTRACTS, vol. 70, no. 17, 28 April 1969, Columbus, Ohio, US; abstract no. 77802r, J.R. GEIGY 'Alkanoic acid N-pyridylamides' page 332 ; & FR-A-1 510 320 (J.R. GEIGY) 19 January 1968**
- **CHEMICAL ABSTRACTS, vol. 70, no. 15, 14 April 1969, Columbus, Ohio, US; abstract no. 68185q, J.R. GEIGY 'N-pyridyl aliphatic carboxylic amides' page 365 ; & FR-A-1 510 319 (J.R. GEIGY) 19 January 1968**
- **CHEMICAL ABSTRACTS. REGISTRY HANDBOOK - NUMBER SECTION. PRINTED ISSUES + MICROFILM COLUMBUS US**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 609 960 B1

Printed by Xerox (UK) Business Services
2.16.7/3.6

## Description

The present invention relates to new N-heteroarylamide derivatives, pharmaceutical compositions comprising such compounds, novel carboxylic acid and acid halide intermediates used in the synthesis of such compounds and the use of such compounds to inhibit intestinal absorption of cholesterol, lower serum cholesterol and reverse the development of atherosclerosis. The compounds are inhibitors of acyl coenzyme A: cholesterol acyltransferase (ACAT).

Cholesterol that is consumed in the diet (dietary cholesterol) is absorbed as free cholesterol by the mucosal cells of the small intestine. It is then esterified by the enzyme ACAT, packaged into particles known as chylomicrons, and released into the bloodstream. Chylomicrons are particles into which dietary cholesterol is packaged and transported in the bloodstream. By inhibiting the action of ACAT, the compounds of this invention prevent intestinal absorption of dietary cholesterol and thus lower serum cholesterol levels. They are therefore useful in preventing atherosclerosis, heart attacks and strokes.

By inhibiting the action of ACAT, the compounds of the present invention also enable cholesterol to be removed from the walls of blood vessels. This activity renders such compounds useful in slowing or reversing the development of atherosclerosis as well as in preventing heart attacks and strokes.

Other inhibitors of ACAT are referred to in United States Patents 4,716,175 and 4,743,605 (a divisional of the '175 patent) and in the European Patent Applications having publication numbers 0 242 610, 0 245 687 and 0 252 524. Certain ureas and thioureas as antiatherosclerosis agents are referred to in United States Patent 4,623,662. US-A-3,555,035 discloses certain N-(substituted pyridyl)-linolamides and -linolenamides having antiviral and tumour inhibiting activity; however none of the compounds are tri-substituted in the pyridine ring, in addition to the amide substituent.

The present invention relates to compounds of the formula

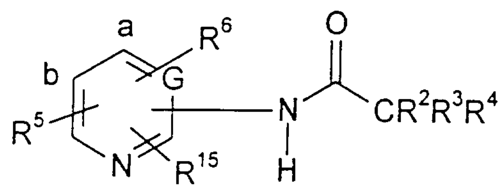

(I)

wherein $R^2$, $R^3$ and $R^4$ are

(a) independently selected from the group consisting of hydrogen, $(C_1\text{-}C_4)$ alkyl, A and $XR^{10}$; with the proviso that at least one of $R^2$, $R^3$ and $R^4$ must be A; or

(b) $R^2$ and $R^3$ together with the carbon atom to which they are attached form a cyclic or bicyclic system selected from the group consisting of $(C_3\text{-}C_7)$ cycloalkyl, $(C_3\text{-}C_7)$ cycloalkenyl, $(C_6\text{-}C_{14})$ bicycloalkyl, $(C_6\text{-}C_{14})$ bicycloalkenyl, and aryl-fused systems containing 8 to 15 carbon atoms, one ring of any of said aryl-fused systems being aromatic and the ring containing the carbon atom to which $R^2$ and $R^3$ are attached being non-aromatic; one of the carbon atoms of said aromatic ring being optionally replaced by sulfur or oxygen; one or more carbon atoms of said non-aromatic ring being optionally replaced by sulfur or oxygen, and one or more carbons of said aromatic ring being optionally replaced by nitrogen; one or two carbon atoms of said cycloalkyl or bicycloalkyl groups being optionally replaced by sulfur or oxygen; and said cyclic or bicyclic system being optionally substituted with one to five substituents independently selected from the group consisting of phenyl, substituted phenyl, $(C_1\text{-}C_6)$ alkyl and A, with the proviso that one and only one of said substituents is A, and one and only one of said substituents is phenyl or substituted phenyl, said substituted phenyl being substituted with one or more substituents independently selected from the group consisting of $(C_1\text{-}C_6)$ alkyl, $(C_1\text{-}C_6)$ alkylthio, halogen and trifluoromethyl; and $R^4$ is hydrogen, $XR^{10}$ or A;

A is a hydrocarbon containing 4 to 16 carbon atoms and 0, 1 or 2 double bonds:
X is S;
$R^5$ is $(C_1\text{-}C_6)$alkylthio;
$R^6$ is $(C_1\text{-}C_6)$ alkylthio;
$R^{15}$ is $(C_1\text{-}C_4)$alkyl or $(C_1\text{-}C_6)$alkylthio;
$R^{10}$ is selected from the group consisting of $(C_4\text{-}C_{12})$ cycloalkyl, $(C_4\text{-}C_{12})$ straight or branched alkyl, $(C_4\text{-}C_{12})$ cycloalkyl-$(C_1\text{-}C_6)$ alkyl, phenyl-$(C_1\text{-}C_6)$alkyl,(substituted phenyl)-$(C_1\text{-}C_6)$ alkyl, $(C_1\text{-}C_6)$ alkyl-phenyl, $(C_1\text{-}C_6)$ alkyl-(substituted phenyl); wherein the substituents on the substituted phenyl, are selected from the group consisting of $(C_1\text{-}C_4)$ alkoxy, $(C_1\text{-}C_4)$ alkylthio, $(C_1\text{-}C_6)$ alkyl, halo and trifluoromethyl:
G is selected from the group consisting of nitrogen and carbon atoms with the proviso that when G is nitrogen,

the resulting pyrimidine group is attached at the 4 or 5 position designated by a and b in formula 1 above; or a pharmaceutically acceptable salt of said compound.

Examples of said aryl-fused and heteroaryl-fused systems are:

1,2,3,4-tetrahydronapthalene,
5,6,7,8,9-pentahydrobenzocycloheptene,
5,6,7,8,9,10-hexahydrobenzocyclooctene,
4,5,6-trihydro-1-thiapentalene,
4,5,6-trihydro-2-thiapentalene,
4,5,6,7-tetrahydrobenzo[b]thiophene,
4,5,6,7-tetrahydrobenzo[c]thiophene,
4,5,6-trihydro-1-oxapentalene,
4,5,6,7-tetrahydrobenzo[b]furan,
4,5,6-trihydro-1-azapentalene,
4,5,6,7-tetrahydrobenzo[b]pyrrole,
4,5,6-trihydro-1-oxa-3-azapentalene,
4,5,6,7-tetrahydrobenzo[d]oxazole,
4,5,6-trihydro-1-thia-3-azapentalene
4,5,6,7-tetrahydrobenzo[d]thiazole,
4,5,6-trihydro-1-oxa-2-azapentalene,
4,5,6,7-tetrahydrobenzo[d]oxazole,
4,5,6-trihydro-1-thia-2-azapentalene,
4,5,6,7-tetrahydrobenzo[d]thiazole,
4,5,6-trihydro-1,2-diazapentalene,
4,5,6,7-tetrahydrobenzo[d]pyrazole,
4,6-diazaindane and
5,6,7,8-tetrahydroquinazoline.

Unless otherwise indicated, the term "halo", as used herein, includes fluoro, chloro, bromo and iodo.
Unless otherwise indicated, the term "alkyl", as used herein, may be straight or branched and may include straight and branched moieties.
Unless otherwise indicated, the term "one or more substituents", as used herein, refers to from one to the maximum number of substituents possible based on the number of available bonding sites.
The term "one or more carbon atoms of said non-aromatic ring", as used herein, refers to from one to all of the carbon atoms that are part of the non-aromatic ring of any of the aryl-fused or heteroaryl-fused systems described above, and not part of the aromatic ring of said aryl-fused system.
The term "one or more carbon atoms of said aromatic ring", as used herein, refers to from one to all of the carbon atoms that are part of the aromatic ring of any of the aryl-fused and heteroaryl-fused systems described above, or are part of both said aromatic and non-aromatic rings of said aryl-fused and heteroaryl-fused system.
The compounds of formula I may have optical centers and therefore may occur in different stereoisomeric configurations. The invention includes all stereoisomers of such compounds of formula I, including mixtures thereof.
Preferred compounds of formula I are those wherein G is N and the resulting group is 2-methyl-4,6-di(methylthio)pyrimidin-5-yl, or G is CH and the resulting group is 6-methyl-2, 4-di(methylthio) - pyridin-3-yl.
Other preferred compounds of formula I are those wherein:

$R^2$ is hexylthio, $R^3$ is octyl and $R^4$ is hydrogen; or
$R^2$ and $R^3$ together with the carbon to which they are attached form an indan-2-yl ring, and $R^4$ is 2-decyl; or
$R^2$ and $R^3$ together with the carbon to which they are attached form a 1,2,3,4-tetrahydronaphth-2-yl ring and $R^4$ is nonyl.

Specific preferred compounds of formula I are:

(2S)-N-[2,4-bis(methylthio)-6-methylpyridin-3-yl]-2-hexylthiodecanoic amide;
(2S)-N-[2-methyl-4,6-bis(methylthio)pyrimidin-5-yl]-2-hexylthiodecanoic amide;
N-[4,6-bis(methylthio)-2-methylpyrimidin-5-yl]-2-hexylthiodecanoic amide;
N-[2,4-bis(methylthio)-6-methylpyridin-3-yl]-2-hexylthiodecanoic amide;
N-[4,6-bis(methylthio)-2-methylpyrimidin-5-yl]-2,2-dimethyldocecanoic amide;

N-[2,4-bis(methylthio)-6-methylpyridin-3-yl]-2,2-dimethyldodecanoic amide;
N-[4,6-bis(methylthio)-2-methylpyrimidin-5-yl]-4,5-dimethyl-trans-2-heptylcyclohex-4-ene-carboxamide;
N-[4,6-bis(methylthio)-2-methylpyrimidin-5-yl]-2-heptylnonanoic amide;
N-[4,6-bis(methylthio)-2-methylpyrimidin-5-yl]pentadecanoic amide;
N-[2,4-bis(methylthio)-6-methylpyridin-3-yl-]pentadecanoic amide;
N-[2,4-bis(methylthio)-6-methylpyridin-3-yl]-(Z)-9-octadecenoic amide;
N-[4,6-bis(methylthio)-2-methylpyrimidin-5-yl]-(Z)-9-octadecenoic amide;
N-[4,6-bis(methylthio)-2-methylpyrimidin-5-yl]-trans-3-nonyl-1,2,3,4-tetrahydro-2-naphthoic amide;

A further compound of formula I is:

N-(2,4-dimethylthio-6-methylpyrimidin-5-yl)-2-heptylnonanoic amide.

The present invention also relates to a pharmaceutical composition for inhibiting ACAT, inhibiting intestinal absorption of cholesterol, reversing or slowing the development of atherosclerosis, or lowering the concentration of serum cholesterol in a mammal, including a human, comprising an amount of a compound of the formula I, or a pharmaceutically acceptable salt thereof, effective in inhibiting ACAT, inhibiting intestinal absorption of cholesterol, reversing or slowing the development of atherosclerosis, or lowering the concentration of serum cholesterol, and a pharmaceutically acceptable carrier.

Examples of pharmaceutically acceptable acid addition salts of the compounds of formula I salts are the salts of hydrochloric acid, p-toluenesulfonic acid, fumaric acid, citric acid, succinic acid, salicyclic acid, oxalic acid, hydrobromic acid, phosphoric acid, methanesulfonic acid, tartaric acid, di-p-toluoyl tartaric acid, and mandelic acid.

Reaction schemes 1-4 below illustrate the synthesis of the compounds of this invention. The compounds of formula I designated in the reaction schemes by the formulae IA, IB, IC, and ID, depending on the method by which they prepared.

Except where otherwise stated, $R^2$, $R^3$, $R^4$, $R^5$, $R^6$, $R^{10}$, $R^{15}$, p, X, A and G in the reaction schemes and discussion that follows are defined as above, p is o, 1 or 2, and $R^1$ is the group:

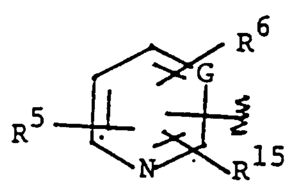

SCHEME 1

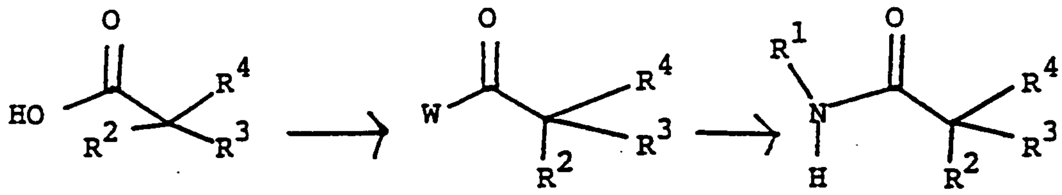

III IV IA

SCHEME 2

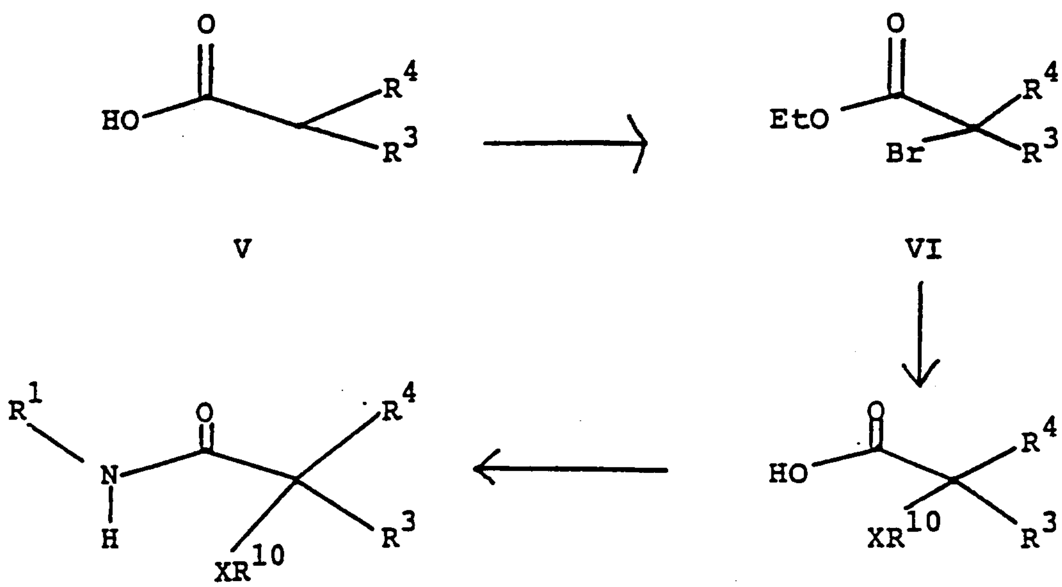

V VI

IB VII

SCHEME 3

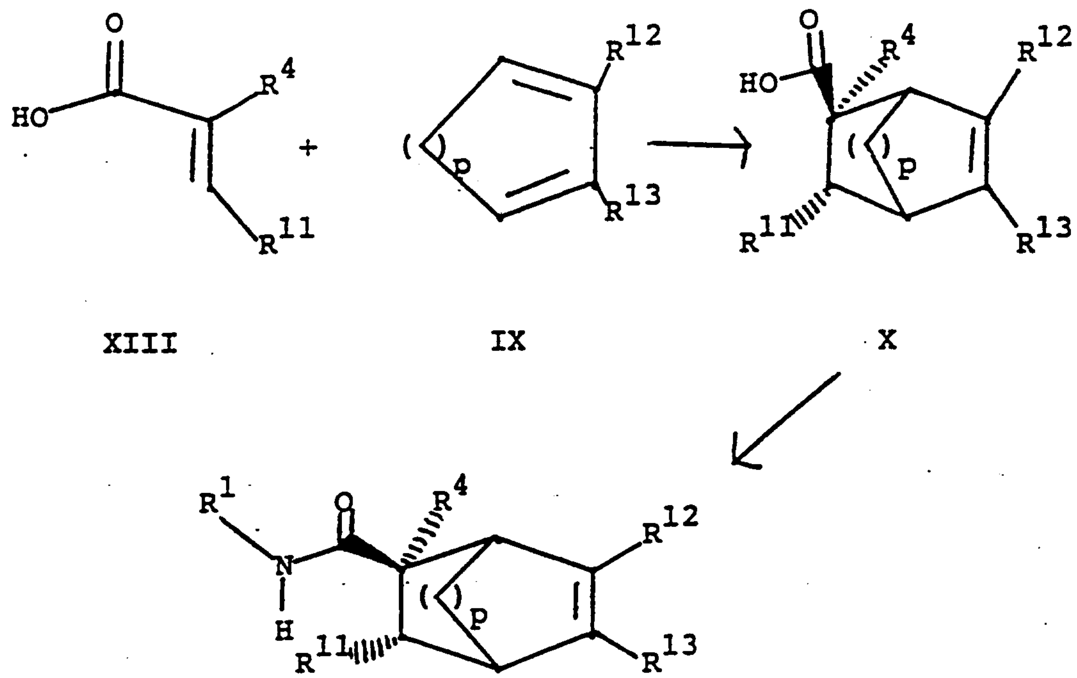

XIII IX X

IC

SCHEME 4

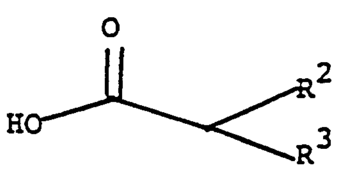

XI

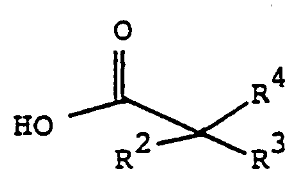

XII

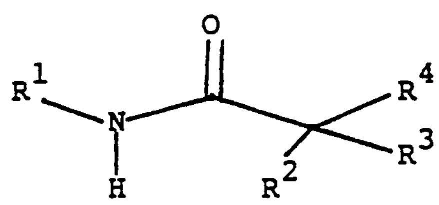

ID

Scheme 1 represents the synthesis of amides of the present invention having the formula IA, from the corresponding carboxylic acid having the formula III. An acid of formula III is first converted to the corresponding acid halide of formula IV, wherein W is chloro or bromo, by reacting it with a chlorinating or brominating agent. Examples of suitable chlorinating and brominating agents are oxalyl chloride, oxalyl bromide, thionyl chloride, thionyl bromide, phosphorous

trichloride, phosphorous tribromide, phosphorous pentachloride, phosphorous pentabromide, phosphorous oxychloride, and phosphorous oxybromide. This reaction is typically carried out in the absence of a solvent or, alternatively, in the presence of a halogenated hydrocarbon solvent such as methylene chloride, for from about .5 to 48 hours (preferably from about 2 to 18 hours) at a temperature from about 0-250°C (preferably at the reflux temperature of the reaction mixture). The acid halide so formed is then converted to the corresponding amide of the formula IA by reacting it with an amine of the formula $R^1NH_2$ and an acid scavenger such as dimethylaminopyridine, pyridine or triethylamine. This reaction is typically carried out in the absence of a solvent or in the presence of an inert solvent such as tetrahydrofuran or methylene chloride for from about 0.25 to 144 hours (preferably from about 2 to 72 hours) at a temperature from about -78 to 350°C (preferably from about -20 to the reflux temperature of the reaction mixture).

Compounds of the formula I, wherein $R^2$ is $XR^{10}$, one of $R^3$ and $R^4$ is hydrogen and the other is A, i.e., compounds of the formula IB, may be prepared as illustrated in scheme 2. Referring to scheme 2, a carboxylic acid of the formula V, wherein one of $R^3$ and $R^4$ is hydrogen and the other is selected from hydrogen, $(C_1\text{-}C_4)$ alkyl or A, is reacted for about 3 hours with thionyl chloride using no solvent, at the reflux temperature of the reaction mixture. Bromine and a catalytic amount of iodine are then added to the reaction mixture, and the resulting mixture is brought to reflux. After refluxing for about 18 hours, ethanol is added and the mixture is refluxed for about 1 more hour to produce a bromoester of the formula VI, wherein $R^3$ and $R^4$ are defined as they are for formula V above. The bromoester of formula VI is then converted to an ester having the same formula as formula VI except that the substituent -Br is replaced by the substituent $-XR^{10}$, (hereinafter referred to as formula VI'), by reacting it with a compound of the formula $HXR^{10}$ and a base such as potassium carbonate or sodium hydride in an aprotic, polar solvent such as dimethylformamide, acetone or tetrahydrofuran, for about .5 to 48 hours (preferably from about 4 to 18 hours) at a temperature from -about -78 to 350°C (preferably from about 0°C to the reflux temperature of the reaction mixture). An acid of the formula VII, wherein $R^3$ and $R^4$ are defined as they are for formulas V and VI above, is then prepared by reacting the ester having formula VI' with a hydroxide such as sodium hydroxide. This reaction is typically carried out overnight in an lower alcohol solvent such as methanol or ethanol, at a temperature from about -78 to 350°C (preferably from about 20°C to the reflux temperature of the reaction mixture).

The acid of formula VII so prepared is then converted to an amide of the formula IB, wherein $R^3$ and $R^4$ are defined as they are for formulae V, VI and VII above, by the acid to amide synthesis illustrated in scheme 1 and described above.

Compounds of the formula IB, may be prepared, alternatively, by the following method. A compound of the formula V, as illustrated in scheme 2 and defined above, is reacted with thionyl chloride followed by bromine and a catalytic amount of iodine as described above, but quenching the reaction with water instead of ethanol, to form a compound of the formula $HOOCCBrR^3R^4$, wherein $R^3$ and $R^4$ are defined as they are for formula V. This compound is then converted, sequentially, to the corresponding acid chloride of the formula $ClCOCBrR^3R^4$ and the corresponding amide of the formula $R^1NHCOCBrR^3R^4$, wherein $R^3$ and $R^4$ are defined as they are for formula V, by the acid to amide synthesis illustrated in scheme 1 and described above. The amide of the formula $R^1NHCOCBrR^3R^4$ so formed is then reacted with a compound of the formula $HXR^{10}$ and a base such as potassium carbonate and sodium hydride to form a compound having the formula IB, wherein $R^3$ and $R^4$ are defined as they are for formula V. This reaction is typically carried out in an aprotic, polar solvent such as dimethylformamide, acetone or tetrahydrofuran, for from about 0.5 to 48 hours (preferably from about 4 to 18 hours). The reaction may be carried out at temperatures ranging from about -78 to 350°C (preferably from about 0°C to the reflux temperature of the reaction mixture). Scheme 3 illustrates the preparation of compounds

of formula I, wherein $R^4$ is hydrogen or A, and $R^2$ and $R^3$, together with the carbon to which they are attached, form the bicyclic ring system

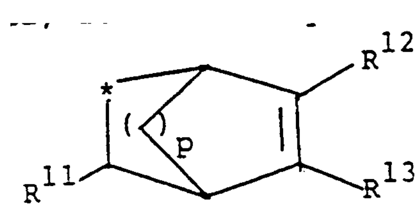

wherein the asterisk designates the carbon to which $R^2$ and $R^3$ are attached, and each of $R^{12}$ and $R^{13}$ are independently selected from the group consisting of hydrogen and $(C_1\text{-}C_4)$ alkyl, or $R^{12}$ and $R^{13}$, together with the carbons to which they are attached, form a benzene ring.

As illustrated in scheme 3, a Diels-Alder reaction is carried out between an acid of the formula XIII, wherein $R^{11}$ is A, hydrogen, phenyl or substituted phenyl, and wherein $R^{11}$ and the carboxyl group are trans to each other, and a diene of the formula IX, wherein $R^{12}$ and $R^{13}$ are as defined above. This reaction is typically carried out in a hydrocarbon solvent such as toluene, using a catalytic amount of an antioxidant such as hydroquinone. The reagents are generally reacted for about 1 to 10 days (preferably for about 3 to 5 days) in a sealed, high pressure apparatus at a temperature from about room temperature to 350°C (preferably from about 100 to 150°C). The reaction yields an acid of the formula X, which can be converted to the corresponding amide of the formula IC, wherein the carbons to which $R^{12}$ and $R^{13}$ are attached are bonded by a carbon-carbon double bond, by the acid to amide synthesis illustrated in scheme 1 and described above. The amide of formula IC so formed can be converted to an amide of the formula IC, wherein the carbons to which $R^{12}$ and $R^{13}$ are attached are bonded by a carbon-carbon single bond, by reacting it with a reducing agent such as hydrogen. Typically, the reduction is carried out using hydrogen gas in a high pressure apparatus, in an inert solvent such as acetic acid, and in the presence of a hydrogenation catalyst such as palladium on carbon.

The reduction maybe carried out at temperatures ranging from about -20 to 250°C (preferably at room temperature). 3.45 bar (fifty p.s.i. of hydrogen) is the preferred pressure, through pressures greater than or equal to 1 atmosphere are suitable. The corresponding compound of formula IC, wherein the carboxyl group and $R^{11}$ are cis to each other, may be prepared in a similar manner, but using the corresponding cis isomer of the acid of formula XIII.

When the procedure of scheme 3 described above is used to prepare a compound of formula IC wherein $R^{12}$ and $R^{13}$, together with the carbon to which they are attached, form a benzene ring, the diene of formula IX is generally generated in situ in the presence of the acid of formula XIII or its ester by heating a mixture of 1,3-dihydrobenzo[c]thiophene 2,2-dioxide and such acid or ester. This reaction is typically carried out at a temperature of from about 235 to 300°C (preferably from about 250 to 265°C) under nitrogen for approximately from 0.5 to 24 hours (preferably for about 2 hours).

Scheme 4 illustrates the preparation of compounds of the formula I, wherein $R^4$ is ($C_1$-$C_4$) alkyl or A, and $R^2$ and $R^3$ are each independently selected from the group consisting of hydrogen, ($C_1$-$C_4$) alkyl, A or $XR^{10}$; or $R^2$ and $R^3$, together with the carbon to which they are attached, form a cyclic or bicyclic system selected from the group consisting of ($C_3$-$C_7$) cycloalkyl, ($C_6$-$C_{14}$) bicycloalkyl, one or two carbons of said cycloalkyl and bicycloalkyl groups being optionally replaced with oxygen or sulfur; and aryl-fused and heteroaryl-fused systems containing 8 to 15 carbon atoms, one ring of any of said aryl-fused and heteroaryl-fused systems being aromatic and the ring containing the carbon to which $R^2$ and $R^3$ are attached being non-aromatic, one of the carbons of said aromatic ring being optionally replaced by sulfur or oxygen, one or more carbons of said non-aromatic ring being optionally replaced by sulfur or oxygen, and one or more carbons of said aromatic ring being optionally replaced by nitrogen. It also illustrates the preparation of compounds of the formula I, wherein $R^4$ is $XR^{10}$, and $R^2$ and $R^3$, together with the carbon to which they are attached, form a cyclic or bicyclic system as defined immediately above. All compounds of the invention illustrated in scheme 4 are designated by the formula ID and are prepared by the following procedure.

An acid of the formula XI, wherein $R^2$ and $R^3$ are each independently selected from the group consisting of hydrogen, ($C_1$-$C_4$) alkyl, A or $XR^{10}$, or $R^2$ and $R^3$ together with the carbon to which they are attached, form a cyclic or bicyclic system as defined immediately above, is reacted with a base such as lithium diisopropylamide or hexamethyldisilazide, with or without an additive such as hexamethylphosphorous triamide, in a dry inert solvent such as tetrahydrofuran, and then reacted with a compound of the formula $R^4$Hal, wherein Hal is halogen and $R^4$ is ($C_1$-$C_7$) alkyl or A. The reaction is typically carried out at a temperature from about -78 to 40°C (preferably from about -78°C to room temperature) for about 0.5 to 48 hours (preferably for about 1.5 to 17 hours: 0.5 to 1 hour to generate the dianion of formula XI and 1 to 16 hours for the alkylation). The product of the reaction is an acid of the formula XII, wherein $R^2$ and $R^3$ are as defined immediately above, and $R^4$ is ($C_1$-$C_7$) alkyl or A. The acid of formula XII so formed may be converted to the corresponding amide of the formula ID, wherein $R^2$ and $R^3$ are as defined immediately above and $R^4$ is ($C_1$-$C_7$) alkyl or A, by the acid to amide synthesis illustrated in scheme 1 and described above.

Compounds of the formula ID, wherein $R^4$ is $XR^{10}$, are prepared by the same procedure as that described above for the preparation of compounds of the formula ID wherein $R^4$ is ($C_1$-$C_7$) alkyl or A, with one modification. The dianion of formula XI is reacted with a compound of formula $R^{10}SSR^{10}$ instead of Hal$R^4$. This reaction produces an acid of the formula XII, wherein $R^4$ is $XR^{10}$. The acid of the formula XII so formed can then be converted to the corresponding amide of formula ID by the acid to amide synthesis illustrated in scheme 1 and described above.

The aminopyrimidine and aminopyridine intermediates used in the present invention are known in the literature or may be prepared by methods known in the art from intermediates that are known in the literature or commercially available. References for the preparation of many of the pyrimidine and pyridine intermediates can be found in the monographs "The Pyrimidines", ed. by D.J. Brown (1962) and "Pyridine and its Derivatives", ed. by R.A. Abramovitch (1961), Interscience Publishers, Inc., New York, N.Y., and their supplements. The preparation of certain of these intermediates is described in greater detail below.

2,6-Disubstituted-5-amino-pyrimidine derivatives may be prepared by reacting the appropriately substituted 4,6-dihydroxypyrimidine with a nitrating agent such as fuming nitric acid in acetic acid at a temperature from about 15°C to about 40°C for a period of about 1 to about 5 hours. The resulting 5-nitropyrimidine is converted to the 2,4-dichloro-5-nitropyrimidine intermediate using a chlorinating agent such as phosphoryl chloride, alone or in the presence of a base, preferably diethylaniline, at a temperature from about 100 to about 115°C for a period of about 0.5 to about 2

hours. Procedures for carrying out these transformations are described in J. Chem. Soc., 3832 (1954).

The 2,6-bis(alkylthio)-5-nitropyrimidine derivatives may be prepared by reacting the appropriate dichloro intermediate with two equivalents of sodium alkylthiolate in a solvent such as dimethylformamide or, preferably, methanol, for about 4 to about 16 hours at a temperature from about 0 to about 30°C, preferably at ambient temperature. Monosubstitution of the dichloro intermediate is then accomplished by using one equivalent of nucleophile, at a reaction temperature of about 0 to about 100°C, depending on the reactivity of the nucleophile, in an inert solvent such as dimethylformamide or tetrahydrofuran, for a period of about 4 to about 16 hours.

The resulting monochloro derivative is then reacted with one equivalent of a different nucleophile to yield a disubstituted derivative with different substitutents on the carbon atoms at positions 2 and 4. The 2,6-disubstituted-5-nitropyrimidine is reduced using a reducing agent such as stannous chloride in concentrated hydrochloric acid or hydrogen gas with an appropriate catalyst, to yield the corresponding 5-aminopyrimidine derivative.

The novel pyridines and other 2,4-disubstituted-3-aminopyridine derivatives may be prepared by reacting the appropriate 2,4-dihydroxypyridine with a nitrating agent such as concentrated nitric acid at 80-100°C for 15-60 minutes. For example, the preparation of 2,4-dihydroxy-6-methyl-3-nitropyridine is described in J. Heterocyclic Chem., 1970, 7, 389. The resulting 2,4-dihydroxy-3-nitropyridine is sequentially converted to the 2,4-dichloro-3-nitropyridine, 2,4-disubstituted-3-nitropyridine and 2,4-disubstituted-3-aminopyridine derivatives, using reaction conditions similar to those described above for the pyrimidine series.

Except where otherwise noted, pressure is not critical in any of the above reactions. Preferred temperatures for the above reactions were stated where known. In general, the preferred temperature for each reaction is the lowest temperature at which product will be formed. The preferred temperature for a particular reaction may be determined by monitoring the reaction using thin layer chromatography.

The novel compounds of formula I and the pharmaceutically acceptable salts thereof are useful as inhibitors of acyl coenzyme A: cholesterol acyltransferase (ACAT). As such they inhibit intestinal absorption of cholesterol in mammals and are useful in the treatment of high serum cholesterol in mammals, including humans. As used herein, treatment is meant to include both the prevention and alleviation of high serum cholesterol. The compound may be administered to a subject in need of treatment by a variety of conventional routes of administration, including orally, parenterally and topically. In general, these compounds will be administered orally or parenterally at dosages between about 0.5 and about 30 mg/kg body weight of the subject to be treated per day, preferably from about .08 to 5 mg/kg. For an adult human of approximately 70 kg of body weight, the usual dosage would, therefore, be about 3.5 to about 2000 mg per day. However, some variation in dosage will necessarily occur depending on the condition of the subject being treated and the activity of the compound being employed. The person responsible for administration will, in any event, determine the appropriate dose for the individual subject.

A compound of formula I or a pharmaceutically acceptable salt thereof may be administered alone or in combination with pharmaceutically acceptable carriers, in either single or multiple doses. Suitable pharmaceutical carriers include inert solid diluents or fillers, sterile aqueous solution and various organic solvents. The resulting pharmaceutical compositions are then readily administered in a variety of dosage forms such as tablets, powders, lozenges, syrups, injectable solutions and the like. These pharmaceutical compositions can, if desired, contain additional ingredients such as flavorings, binders, excipients and the like. Thus, for purposes of oral administration, tablets containing various excipients such as sodium citrate, calcium carbonate and calcium phosphate may be employed along with various disintegrants such as starch, alginic acid and certain complex silicates, together with binding agents such as polyvinylpyrrolidone, sucrose, gelatin and acacia. Additionally, lubricating agents such as magnesium stearate, sodium lauryl sulfate and talc are often useful for tabletting purposes. Solid compositions of a similar type may also be employed as fillers in soft and hard filled gelatin capsules. Preferred materials for this include lactose or milk sugar and high molecular weight polyethylene glycols. When aqueous suspensions or elixirs are desired for oral administration, the essential active ingredient therein may be combined with various sweetening or flavoring agents, coloring matter or dyes and, if desired, emulsifying or suspending agents, together with diluents such as water, ethanol, propylene glycol, glycerin and combinations thereof.

For parenteral administration, solutions of a compound of formula I or a pharmaceutically acceptable salt thereof in sesame or peanut oil, aqueous propylene glycol, or in sterile aqueous solution may be employed. Such aqueous solutions should be suitably buffered if necessary and the liquid diluent first rendered isotonic with sufficient saline or glucose. Such solutions are especially suitable for intravenous, intramuscular, subcutaneous and intraperitioneal administration. In this connection, the sterile aqueous media employed are all readily available by standard techniques known to those skilled in the art.

The activity of the compounds of the present invention as ACAT inhibitors may be determined by a number of standard biological or pharmacological tests. For example, the following procedure was used to determine the ACAT inhibiting activity of compounds of formula I. ACAT was assayed in microsomes isolated from chow fed Sprague-Dawley rats according to Bilheimer, J.T., Meth. Enzymol., 111, ps 286-293 (1985), with minor modifications. Microsomes from rat liver were prepared by differential centrifugation and washed with assay buffer prior to use. The assay mixture contained 25 ul of BSA (40 mg/ml), 30 ul of rat liver microsome solution (100 ug microsomal protein), 20 ul of assay buffer (0.1 M $K_2PO_4$, 1.0 mM reduced Glutathione, pH 7.4), 20 ug of cholesterol in 100 ul of a 0.6% Triton WR-1339 solution in assay buffer, and 5 ul of test compound dissolved in 100% DMSO (total volume = 180 ul). The assay mixture was incubated for 30 min at 37°C. The reaction was started by the addition of 20 ul of 14C-Oleoyl-CoA (1000 uM, 2,000 dpm/nmol) and run for 15 min at 37°C. The reaction was stopped by the addition of 1 ml ETOH. The lipids were extracted into 4 ml hexane. A 3 ml aliquot was dried under $N_2$, and resuspended in 100 ul of chloroform. 50 ul of chloroform were spotted on a heat activated TLC plate and developed in hexane: diethyl ether: acetic acid (85:15:1, v:v:v). Incorporation of radioactivity into cholesteryl esters was quantified on a Berthold LB2842 Linear TLC Analyzer. ACAT inhibition was calculated relative to a DMSO control assay.

The activity of the compounds of formula I in inhibiting intestinal absorbtion of cholesterol may be determined by the procedure of Melchoir and Harwell, J. Lipid. Res., 26, 306-315 (1985).

The present invention is illustrated by the following examples. It will be understood, however, that the invention is not limited to the specific details of these examples. Melting points are uncorrected. Proton nuclear magnetic resonance spectra ($^1H$ NMR) and $C^{13}$ nuclear magnetic resonance spectra ($C^{13}$ NMR) were measured for solutions in deuterochoroform ($CDCl_3$) and peak positions are expressed in parts per million (ppm) downfield from tetramethylsilane (TMS). The peak shapes are denoted as follows: s, singlet; d, doublet; t, triplet; q, quartet; m, multiplet; br, broad; c, complex.

EXAMPLE 1

Ethyl 2-(4-n-Propylphenylthio)nonanoate

1.6 g (0.033 mole) sodium hydride (50% dispersion in mineral oil) was added to a solution of 5.0 g (0.033 mole) 4-propylthiophenol in 25 ml anhydrous dimethylformamide. After 15 minutes, 8.8 g (0.033 mole) ethyl 2-bromononanoate (prepared according to J. Labelled Compounds Radiopharm. 14, 713 (1978)) was added and the resulting mixture was stirred at room temperature overnight. The reaction mixture was then diluted with 150 ml ethyl acetate and the resulting mixture was washed with 5 x 60 ml water and then with 60 ml saturated aqueous sodium chloride solution. The ethyl acetate solution was dried over anhydrous sodium sulfate, filtered and concentrated in vacuo. The resulting oil was chromatographed on 600 g silica gel, eluting with 7:3 hexane/methylene chloride to yield 9.0 g (81% yield) of the desired product as an oil.

$^1H$ NMR($CDCl_3$): $\delta$ 0.88 (c,6H); 1.1-1.5 (c,total 12H) including 1.12 (t,3H); 1.54-1.93 (c,4H); 2.54 (t,2H); 3.56 (q,1H); 4.07 (q,2H); 7.1 (d,2H); 7.36 (d,2H).

EXAMPLE 1A

2-Hexylthiodecanoic Acid

17.3 g (0.36 mol) sodium hydride (50% dispersion in mineral oil) was added portionwise with stirring (gas evolution) to a solution of 26.8 ml. (0.19 mol) hexanethiol in 500 ml. anhydrous dimethylformamide. The mixture was stirred at toom temperature for 30 min., then 45.2g (0.18 mol) 2-bromodecanoic acid was added dropwise with stirring, keeping the temperature of the reaction mixture below 45°C. The reaction mixture was stirred at room temperature under nitrogen overnight. The mixture was then diluted with 500 ml. water and the pH of the resulting mixture was adjusted to 1.5 with 6N aqueous hydrochloric acid solution.

This mixture was extracted with 3 x 400 ml. ethyl acetate and the combined ethyl acetate extracts were washed with 5 x 700 ml. water and 1 x 500 ml. brine. The ethyl acetate solution was dried over anhydrous sodium sulfate, filtered and concentrated in vacuo. The resulting oil was chromatographed on 2 kg. silica gel, eluting with methylene chloride to yield 35g (67% yield) of the desired product as an oil.

EXAMPLE 1B

Resolution of 2-hexylthiodecanoic acid

2-Hexylthiodecanoyl chloride was prepared by the procedure of Example 4A. A solution of 2-hexylthiodecanoyl chloride (2.39 g., 7.8 mmol) in 20 ml methylene chloride was added slowly with stirring under nitrogen to a solution of (R)-(-)-2-phenylglycinol (1.08 g, 7.9 mmol) and 4-dimethylaminopyridine (0.96 g, 7.9 mmol) in 80 ml methylene chloride at 5°C. The reaction mixture was stirred at room temperature overnight. Methylene chloride (100 ml.) was then added and the resulting solution was washed sequentially with 100 ml 1N aqueous hydrochloric acid solution, 100 ml water,

100 ml saturated aqueous sodium bicarbonate solution and 100 ml brine. The organic phase was dried over anhydrous sodium sulfate and concentrated in vacuo to a solid residue (3.1 g). The diastereomers were separated by column chromatography on 800g silica gel using 1:1 hexane-diethyl ether as eluant. The less polar diastereomer (1.09 g, $[\alpha]_D^{RT}$ = -9.85° ($CH_3OH$); mp 98-100°C) and 0.99 g of the more polar diastereomer ($[\ ]_D^{RT}$ = -9.46°($CH_3OH$); mp 105-108°C) were obtained along with 0.36 g of a mixture of diastereomers (total yield 76%). A solution of the less polar diastereomer (900 mg, 2.2 mmol) in 42 ml 1,4-dioxane and 42 ml 6N aqueous sulfuric acid solution was heated at 105°C under nitrogen for 15 hours. The reaction mixture was cooled to room temperature, diluted with 80 ml water and the resulting mixture was extracted with 4 x 60 ml ethyl acetate. The combined ethyl acetate extracts were washed with 60 ml brine, dried over anhydrous sodium sulfate and concentrated in vacuo to yield (S)-(-)-2-hexylthiodecanoic acid as an oil (634 mg., 99.6% yield); $[\alpha]_D^{RT}$ = -59.5° ($CH_3OH$).

In a similar manner, hydrolysis of the more polar diastereomer yielded 98.4% of (R)-(+)-2-hexylthiodecanoic acid as an oil; $[\alpha]_D^{RT}$ = +54.0°($CH_3OH$).

EXAMPLE 2

Ethyl 2-(4-t-Butylphenylthio)octanoate

A mixture of 5.0 g (0.02 mole) ethyl 2-bromooctanoate, 3.37 g (0.02 mole) p-t-butylthiophenol and 3.31 g (0.24 mole) potassium carbonate in 70 ml acetone was refluxed under nitrogen overnight. The reaction mixture was cooled to room temperature and filtered and the filtrate was concentrated in vacuo. The residue was chromatographed on 500 g silica gel, eluting with 6:4-methylene chloride/hexane to yield 3.8 g (57% yield) of the desired product as an oil.

$^1H$ NMR($CDCl_3$): δ 0.88 (c,3H); 1.1-1.52 (c,total 20H) including 1.14 (t,3H) and 1.3 (s); 1.66-2.11 (c,2H); 358 (q,1H); 4.1 (q,2H); 7.36 (m,4H).

EXAMPLE 3

2-(4-n-Propylphenylthio)nonanoic acid

A solution containing 5.7 (0.017 mole) of the title compound of Example 1, 35 ml of 1N aqueous sodium hydroxide solution (0.035 mole) and 3 ml methanol was refluxed overnight. The resulting solution was cooled to room temperature, acidified to pH 1.5 with 2N aqueous hydrochloric acid and extracted with 3 x 50 ml ethyl acetate. The combined ethyl acetate extracts were washed with 50 ml water and 50 ml saturated aqueous sodium chloride solution, dried over anhydrous sodium sulfate and concentrated in vacuo to yield the title compound as an oil (5.0 g, 96% yield) which was used in the subsequent reaction without further purification.

$^1H$ NMR($CDCl_2$): δ 0.88 (c, 6H); 1.17-1.54 (c, 12H); 1.54-1.92 (c, 4H); 2.53 (t, 2H); 3.54 (t, 1H); 7.1 (d, 2H); 7,37 (d, 2H).

EXAMPLE 4 (ILLUSTRATIVE)

2-(4-n-Propylphenylthio)-N-(2,4,6-trimethoxyphenyl)non-anamide

1.54 g (5 mmole) of the title compound of Example 3 in 20 ml of thionyl chloride was refluxed for 3 hours and then concentrated to dryness in vacuo. 523 mg (1.6 mmole) of the resulting acid chloride was dissolved in 20 ml methylene chloride and to the solution was added 292 mg (1.6 mmole) 2,4,6-trimethoxyaniline and 195 mg (1.6 mmole) 4-dimethylaminopyridine. The resulting solution was stirred at room temperature overnight and then concentrated in vacuo. The residue was partitioned between 60 ml ethyl acetate and 20 ml 1N aqueous hydrochloric acid solution. The ethyl acetate layer was washed with 50 ml water and 50 ml saturated aqueous sodium chloride solution, dried over anhydrous sodium sulfate and concentrated in vacuo. The crude product was chromatographed on 100 g silica gel, eluting with 1:1 hexane/ethyl acetate to yield 370 mg (49% yield) of the title compound as a whitish solid.

EXAMPLE 4A

N-[2-methyl-4,6-bis(methylthio) pyrimidin-5-yl]-2-hexylthiodecanoic amide

A solution of 6.49 g (22.5 mmol) 2-hexylthiodecanoic acid in 40 ml thionyl chloride and 100 ml benzene was refluxed under nitrogen for 2.5 hours and then concentrated to dryness in vacuo. The resulting acid chloride (6.88 g, 22.5 mmol) was dissolved in 15 ml. methylene chloride and the solution was added dropwise to a solution of 4.63 g (23 mmol) 5-amino-4,6-bis(methylthio)-2-methylpyrimidine in 140 ml methylene chloride. The resulting solution was

refluxed under nitrogen overnight. The reaction solution was then cooled, diluted with 140 ml methylene chloride and washed with 2 x 125 ml 3N aqueous hydrochloric acid solution, 1 x 125 ml water, 1 x 125 ml saturated aqueous sodium bicarbonate solution and 1 x 125 ml brine. The methylene chloride solution was dried over anhydrous sodium sulfate, filtered and concentrated to dryness in vacuo. The solid residue was recrystallized from diethyl ether yielding 5.35 g of the title compound, m.p. 99-101°C. The filtrate was concentrated in vacuo and the residue was chromatographed on 400 g silica gel eluting with 9:1 hexane/ethyl acetate. Recrystallization of the product obtained by chromatography from diethyl ether yielded another 2.32 g of the title compound, m.p. 99°-101°C (total yield 72.4%).

$^1$H NMR ($CDCl_3$): δ 0.87 (c, 6H); 1.21-1.84 (c, 21 H), 2.02 (m, 1H); 2.50 (s, 6H); 2.76 (s, 3H), 2.74 (t, 2H); 3.45 (t, 1H), 8.08 (s, 1H);
IR ($CHCl_3$): 2923, 2852, 1681, 1511, 1468, 1431, 1405 $cm^{-1}$.

EXAMPLE 4B

N-[2,4-bis(methylthio)-6-methylpyridin-3-yl]-2-hexylthiodecanoic amide

A solution of 4.19 g (13.7 mmol) 2-hexylthiodecanoyl chloride, prepared according to Example 4A, in 15 ml methylene chloride was added dropwise with stirring under nitrogen to a solution of 2.75 g (13.7 mmol) 3-amino-2,4-bis(methylthio)-6-methylpyridine in 30 ml pyridine cooled to 5°C. The reaction mixture was stirred at room temperature under nitrogen overnight.

Methylene chloride (250 ml) was then added to the reaction mixture and the resulting solution was washed with 3 x 50 ml 3N aqueous hydrochloric acid solution, 2 x 50 ml water, 1 x 50 ml saturated aqueous sodium bicarbonate solution and 1 x 50 ml brine. The methylene chloride solution was dried over anhydrous sodium sulfate, filtered and concentrated to dryness in vacuo.

The solid residue (6.5 g) was recrystallized from petroleum ether to yield 4.7 g of the title compound, m.p. 75-76.5°C (72.8% yield).

$^1$H NMR ($CDCl_3$): δ 0.86 (c, 6H); 1.16-1.74 (c, 21H); 2.04 (m, 1H); 2.4 (s, 3H); 2.48 (s, 3H); 2.5 (s, 3H); 2.77 (t, 2H); 3.45 (t, 1H); 6.65 (s, 1H); 8.14 (s, 1H).
IR ($CHCl_3$): 2922, 2852, 1676, 1600, 1561, 1466 $cm^{-1}$.

EXAMPLE 5

N-[2,4,6-tris(methylthio)pyrimidin-5-yl]-2-hexylthiodecanoic amide

The title compound was prepared in 79% yield according to the procedure of Example 4A.

$^1$H NMR ($CDCl_3$): δ 0.87 (c, 6H); 1.17-1.86 (c, 21H); 2.01 (m, 1H); 2.50 (s, 6H); 2.56 (s, 3H); 2.73 (t, 2H); 3.43 (t, 1H); 8.06 (s, 1H).
IR ($CHCl_3$): 2922, 2852, 1686, 1499, 1465, 1347 $cm^{-1}$.

EXAMPLE 6

N-[2-methyl-4,6-bis(ethylthio)pyrimidin-5-yl]-2-hexylthiodecanoic amide

The title compound was prepared in 52% yield according to the procedure of Example 4A.

$^1$H NMR ($CDCl_3$): δ 0.87 (m, 6H); 1.19-1.84 (c, 27H); 2.0 (m, 1H); 2.57 (s, 3H); 2.75 (t, 2H); 3.15 (q, 4H); 3.44 (t, 1H); 8.04 (s, 1H).
IR ($CHCl_3$): 2920, 2852, 1680, 1467, 1406, 1359, 1314 $cm^{-1}$.

EXAMPLE 7

N-[2,4-bis(methylthio)-6-methylpyridin-3-yl]-2-heptylnonanoic amide

The title compound was prepared in 48% yield according to the procedure of Example 4B.

$^1$H NMR ($CDCl_3$): δ 0.87 (m, 6H); 1.17-1.82 (c, 24H); 2.28 (m, 1H); 2.4 (s, 3H); 2.48 (s, 3H); 2.50 (s, 3H); 6.53 (s,

1H); 6.63 (s, 1H).
IR ($CHCl_3$): 2921, 2851, 1686, 1560, 1460, 1338 $cm^{-1}$.

EXAMPLE 8

N-[2-methyl-4,6-bis(methylthio)pyrimidin-5-yl]-2-heptylnonanoic amide

The title compound was prepared in 35% yield according to the procedure of Example 4A.

$^1H$ NMR ($CDCl_3$): $\delta$ 0.87 (m, 6H); 1.18-1.8 (c, 24H); 2.27 (m, 1H); 2.49 (s, 6H); 2.59 (s, 3H); 6.46 (s, 1H).
IR ($CHCl_3$): 2920, 2850, 1691, 1505, 1462, 1431, 1406, 1360, 1300 $cm^{-1}$.

EXAMPLE 9

N-[2,4-bis(methylthio)-6-methylpyridin-3-yl]-2,2-dimethyldodecanoic amide

The title compound was prepared in 49% yield according to the procedure of Example 4B.

$^1H$ NMR ($CDCl_3$): $\delta$ 0.87 (t, 3H); 1.18-1.67 (c) and 1.32 (s) (total 24H); 2.39 (s, 3H); 2.48 (s, 3H); 2.50 (s, 3H); 6.63 (s, 1H); 6.72 (s, 1H).
IR ($CHCl_3$): 2920, 2850, 1678, 1559, 1459, 1338 $cm^{-1}$.

EXAMPLE 10

N-[2-methyl-4,6-bis(methylthio)pyrimidin-5-yl]-2,2-dimethyldodecanoic amide

The title compound was prepared in 23% yield according to the procedure of Example 4A.

$^1H$ NMR ($CDCl_3$): $\delta$ 0.86 (t, 3H); 1.2-1.68 (c) and 1.31 (s)(total 24H); 2.49 (s, 6H); 2.59 (s, 3H); 6.65 (s, 1H).
IR ($CHCl_3$): 2923, 2849, 1683, 1510, 1467, 1407, 1362, 1301 $cm^{-1}$.

EXAMPLE 11

N-[2,4-bis(ethylthio)-6-methylpyridin-3-yl]-tetradecanoic amide

The title compound was prepared in 68% yield according to the procedure of Example 4B.

$^1H$ NMR ($CDCl_3$): $\delta$ 0.87 (t, 3H); 1.19-1.62 (c, 26H); 1.76 (m, 2H); 2.39 (t, 2H); 2.46 (s, 3H); 2.91 (q, 2H); 3.15 (q, 2H); 6.52 (s, 1H), 6.68 (s, 1H).
IR ($CHCl_3$): 2920, 2850, 1687, 1556, 1460 $cm^{-1}$.

EXAMPLE 12

N-[2,4-bis(methylthio)-6-methylpyridin-3-yl]-tetradecanoic amide

The title compound was prepared in 59% yield according to the procedure of Example 4B.

$^1H$ NMR ($CDCl_3$): $\delta$ 0.87 (t, 3H); 1.18-1.82 (c, 22H); 2.40 (s), 2.48 (s), 2.50 (s) and 2.37-2.6 (m)(total 11H); 6.50 (s, 1H); 6.64 (s, 1H).
IR ($CHCl_3$): 2917, 2847, 1693, 1570, 1472 $cm^{-1}$.

EXAMPLE 13

N-[2-methyl-4,6-bis(methylthio)pyrimidin-5-yl]tetradecanoic amide

The title compound was prepared in 78% yield according to the procedure of Example 4A.

$^1H$ NMR ($CDCl_3$): $\delta$ 0.87 (t, 3H); 1.19-1.61 (c, 20H); 1.75 (m, 2H); 2.40 (t, 2H); 2.49 (s, 6H); 2.59 (s, 3H); 6.45 (s,

1H).
IR ($CHCl_3$): 2917, 2847, 1689, 1460, 1406, 1357 $cm^{-1}$.

EXAMPLE 14

N-[2-methyl-4,6-bis(methylthio)pyrimidin-5-yl]pentadecanoic amide

The title compound was prepared in 53% yield according to the procedure of Example 4A.

$^1H$ NMR ($CDCl_3$): δ 0.87 (t, 3H); 1.18-1.81 (c, 24H), 2.4 (t, 2H); 2.5 (s, 6H), 2.6 (s, 3H); 6.44 (s, 1H).
IR ($CHCl_3$): 2918, 2847, 1689, 1460, 1425, 1405 $cm^{-1}$.

EXAMPLE 15

N-[2,4-bis(methylthio)-6-methylpyridin-3-yl]pentadecanoic amide

The title compound was prepared in 68% yield according to the procedure of Example 4B.

$^1H$ NMR ($CDCl_3$): δ 0.87 (t, 3H); 1.18-1.82 (c, 24H); 2.40 (s + t, 5H); 2.51 (s, 3H); 6.52 (s, 1H); 6.63 (s, 1H).
IR ($CHCl_3$): 2921, 2849, 1666, 1612, 1559, 1459 $cm^{-1}$.

EXAMPLE 16

N-[2-methyl-4,6-bis(methylthio)pyrimidin-5-yl]hexadecanoic amide

The title compound was prepared in 78.2% yield according to the procedure of Example 4A.

$^1H$ NMR ($CDCl_3$): δ 0.87 (t, 3H); 1.18-1.49 (c, 22H), 1.57 (m, 2H); 1.75 (m, 2H); 2.39 (t, 2H); 2.49 (s, 6H); 2.59 (s, 3H); 6.46 (s, 1H).
IR ($CHCl_3$): 2919, 2849, 1688, 1459, 1406, 1358 $cm^{-1}$.

EXAMPLE 17

N-[2,4-bis(methylthio)-6-methylpyridin-3-yl]hexadecanoic amide

The title compound was prepared in 8.6% yield according to the procedure of Example 4.

$^1H$ NMR ($CDCl_3$): δ 0.87 (t, 3H); 1.18-1.84 (c, 26H); 2.39 (s + t, 5H); 2.48 (s, 3H); 2.5 (s, 3H); 6.5 (s, 1H); 6.64 (s, 1H).
IR ($CHCl_3$): 2921, 2849, 1690, 1612, 1560, 1460 $cm^{-1}$.

EXAMPLE 18

N-[2,4-bis(methylthio)-6-methylpyridin-3-yl]-(Z)-9-octadecenoic amide

The title compound was prepared in 55% yield according to the procedure of Example 4.

$^1H$ NMR ($CDCl_3$): δ 0.87 (t, 3H); 1.18-1.68 (c, 20H); 1.77 (m, 2H); 2.0 (c, 4H); 2.39 (s + t, 5H); 2.47 (s, 3H); 2.49 (s, 3H); 5.34 (m, 2H); 6.51 (s, 1H); 6.63 (s, 1H).
IR ($CHCl_3$): 2918, 2850, 1686, 1560, 1460, 1339 $cm^{-1}$.

EXAMPLE 19

N-[2-methyl-4,6-bis(ethylthio)pyrimidin-5-yl]-(Z)-9-octadecenoic amide

The title compound was prepared in 66.7% yield according to the procedure of Example 4A.

$^1H$ NMR ($CDCl_3$): δ 0.87 (t, 3H); 1.18-1.5 (c, 24H); 1.58 (m, 2H); 1.75 (m, 2H); 2.01 (c, 4H); 2.38(t, 2H); 2.57 (s,

3H), 3.14 (q, 4H); 5.34 (m, 2H); 6.41 (s, 1H).
IR ($CHCl_3$): 2919, 2849, 1690, 1459, 1407, 1357, 1312 $cm^{-1}$.

EXAMPLE 20

N-[2-methyl-4,6-bis(methylthio)pyrimidin-5-yl]-(Z)-9-octadecenoic amide

The title compound was prepared in 55% yield according to the procedure of Example 4.

$^1H$ NMR ($CDCl_3$): δ 0.87 (t, 3H); 1.18-1.48 (c, 18H); 1.58 (m, 2H); 1.76 (m, 2H); 2.0 (c, 4H); 2.39 (t, 2H); 2.49 (s, 6H); 2.59 (s, 3H); 5.33 (m, 2H); 6.46 (s, 1H).
IR ($CHCl_3$): 2923, 2850, 1692, 1508, 1464, 1429, 1406, 1360 $cm^{-1}$.

EXAMPLE 21

N-[2,4-bis(methylthio)-6-methylpyridin-3-yl]-2-dodecylthioacetamide

The title compound was prepared in 45% yield according to the procedure of Example 4.

$^1H$ NMR ($CDCl_3$): δ 0.87 (t, 3H); 1.18-1.46 (c, 18H); 1.67 (m, 2H); 2.41 (s, 3H); 2.49 (s, 3H); 2.51 (s, 3H); 2.76 (t, 2H); 3.41 (s, 2H); 6.66 (s, 1H); 8.25 (s, 1H).
IR($CHCl_3$): 2918, 2848, 1678, 1561, 1476, 1337 $cm^{-1}$.

EXAMPLE 22

N-[2,4-bis(ethylthio)-6-methylpyridin-3-yl]-2-dodecylthioacetamide

The title compound was prepared in 37% yield according to the procedure of Example 4B.

$^1H$ NMR ($CDCl_3$): δ 0.87 (t, 3H); 1.18-1.47 (c, 24H); 1.67 (m, 2H); 2.47 (s, 3H); 2.77 (t, 2H); 2.92 (q, 2H); 3.15 (q, 2H); 3.41 (s, 2H); 6.69 (s, 1H); 8.24 (s, 1H).
IR ($CHCl_3$): 2920, 2850, 1680, 1559, 1474, 1337 $cm^{-1}$.

EXAMPLE 23

N-[2-methyl-4,6-bis(methylthio)pyrimidin-5-yl]-trans-3-nonyl1,2,3,4-tetrahydro-2-naphthoic amide

The title compound was prepared in 7% yield according to the procedure of Example 4A. $^1H$ NMR ($CDCl_3$): δ 0.87 (m, 3H); 1.2-1.72 (c, 16H); 2.16 (m, 1H); 2.41-2.64 (c), 2.51 (s), 2.60 (s)(total 11H); 2.94-3.28 (c, 3H); 6.54 (s, 1H); 7.12 (c, 4H).

IR ($CHCl_3$): 2900, 2830, 1690, 1460 $cm^{-1}$.

EXAMPLE 24

N-[2-methyl-4,6-bis(methylthio)pyrimidin-5-yl]-2-decylcyclonentanecarboxamide

The title compound was prepared in 27.4% yield according to the procedure of Example 4A.

$^1H$ NMR ($CDCl_3$): δ 0.87 (t, 3H); 1.2-1.82 (c, 24H); 2.27 (m, 2H); 2.49 (s, 6H); 2.59 (s, 3H); 6.6 (s, 1H).
IR ($CHCl_3$): 2921, 2851, 1681, 1450, 1407, 1360 $cm^{-1}$.

EXAMPLE 25

N-[2,4-bis(methylthio)-6-methylpyridin-3-yl]-2-methylthiotetradecanoic amide

The title compound was prepared in 66% yield according to the procedure of Example 4B.

$^1H$ NMR ($CDCl_3$): δ 0.87 (t, 3H); 1.2-1.87 (c, 21H); 2.03 (m, 1H); 2.31 (s, 3H); 2.41 (s, 3H); 2.50 (s, 3H); 2.53 (s, 3H), 3.38 (t, 1H); 6.66 (s, 1H); 8.05 (s, 1H).
IR ($CHCl_3$): 2919, 2850, 1677, 1559, 1522, 1468, 1438 $cm^{-1}$.

EXAMPLE 26

N-[2-methyl-4,6-bis(methylthio)pyrimidin-5-yl]-2-methylthiotetradecanoic amide

The title compound was prepared in 79% yield according to the procedure of Example 4A.

$^1H$ NMR ($CDCl_3$): δ 0.87 (t, 3H); 1.21-1.86 (c, 21H); 2.04 (m, 1H), 2.29 (s, 3H); 2.52 (s, 6H); 2.62 (s, 3H); 3.37 (t, 1H); 8.0 (s, 1H).
IR ($CHCl_3$): 2918, 2849, 1681, 1465, 1405 $cm^{-1}$.

EXAMPLE 27

N-[2,4-bis(methylthio)-6-methylpyridin-3-yl]-2-ethylthiotetradecanoic amide

The title compound was prepared in 51% yield according to the procedure of Example 4B.

$^1H$ NMR ($CDCl_3$): δ 0.87 (t, 3H); 1.2-1.7 (c, 23H); 1.8 (m, 1H); 2.06 (m, 1H); 2.41 (s, 3H); 2.50 (s, 3H); 2.53 (s, 3H); 2.8 (q, 2H); 3.48 (t, 1H); 6.66 (s, 1H); 8.13 (s, 1H).
IR ($CHCl_3$): 2920, 2850, 1675, 1560, 1466 $cm^{-1}$.

EXAMPLE 28

N-[2-methyl-4,6-bis(methylthio)pyrimidin-5-yl]-2-ethylthiotetradecanoic amide

The title compound was prepared in 51% yield according to the procedure of Example 4A.

$^1H$ NMR ($CDCl_3$): δ 0.87 (t, 3H); 1.2-1.88 (c, 24H); 2.03 (m, 1H); 2.52 (s, 6H); 2.62 (s, 3H); 2.79 (q, 2H); 3.48 (t, 1H); 8.08 (s, 1H).
IR ($CHCl_3$): 2920, 2850, 1679, 1465, 1405 $cm^{-1}$.

EXAMPLE 29

N-[2-methyl-4,6-bis(methylthio)pyrimidin-5-yl]-4,5-dimethyl-trans-2-heptylcyclohex-4-enecarboxamide

The title compound was prepared in 31% yield according to the procedure of Example 4A.

$^1H$ NMR ($CDCl_3$): δ 0.87 (t, 3H); 1.16-2.48 (c, 24H); 2.5 (s, 6H); 2.59 (s, 3H); 6.56 (s, 1H).
IR ($CHCl_3$): 2918, 2850, 1687, 1458, 1406, 1360 $cm^{-1}$.

EXAMPLE 30

(2S)-N-[2,4-bis(methylthio)-6-methylpyridin-3-yl]-2-hexylthiodecanoic amide

(S)-(-)-2-Hexylthiodecanoic, prepared according to Example IB, was coupled with 3-amino-2,4-bis(methylthio)-6-methylpyridine by the procedure of Example 4B to yield the title compound in 55% yield; $[\alpha]_D^{RT}$ = -59° ($CH_3OH$). A sample recrystallized from petroleum ether had mp 81-83°C and $[\alpha]_D^{RT}$ = -66° ($CH_3OH$).

EXAMPLE 31

(2R)-N-[2,4-bis(methylthio)-6-methylpyridin-3-yl]-2-hexylthiodecanoic amide

The title compound was prepared in 30.1% yield according to a procedure similar to that of Example 71. A sample recrystallized from petroleum ether had mp 80-82°C and $[\alpha]_D^{RT}$ = +61.7° ($CH_3OH$).

EXAMPLES 32

(2S)-N-[2-methyl-4,6-bis(methylthio)pyrimidin-5-yl]-2-hexylthiodecanoic amide

The title compound was prepared in 47.2% yield by the coupling of S-(-)-2-hexylthiodecanoic acid with 5-amino-4,6-bis(methylthio)-2-methylpyrimidine according to the procedure of Example 4A. A sample recrystallized from diethyl ether had mp 98-100°C and $[\alpha]_D^{RT}$ = -62° ($CH_3OH$).

EXAMPLE 33

(2R)-N-[2-methyl-4,6-bis(methylthio)pyrimidin-5-yl]-2-hexylthiodecanoic amide

The title compound was prepared in 50.3% yield by a procedure similar to that of Example 73. A sample recrystallized from diethyl ether had mp 95-97.5°C and $[\alpha]_D^{RT}$ = +56.0° ($CH_3OH$).

EXAMPLE 34 (INTERMEDIATE)

3-Amino-2,4-bis(methylthio)-6-methylpyridine

To a solution of 15.5 g (0.22 mol) sodium methanethiolate in 200 ml methanol was added slowly with stirring under nitrogen a solution of 20.8 g (0.1 mol) 3-nitro-2,4-dichloro-6-methylpyridine in 150 ml methanol. A precipitate formed and the mixture was stirred overnight at room temperature. The mixture was then filtered and the solid was washed first with methanol and then with water. 3-Nitro-2,4-bis(methylthio)-6-methylpyridine (18.9 g, 82% yield) was obtained as a yellow solid, mp 172-176°C.

$^1$H NMR ($CDCl_3$): δ 2.45 (s, 3H); 2.51 (s, 3H); 2.55 (s, 3H); 6.77 (s, 1H).

A mixture of 18.9 g (0.082 mol) 3-nitro-2,4-bis(methylthio)-6-methylpyridine and 18.9 g Raney nickel in 600 ml. 1,4-dioxane and 300 ml methanol was shaken with hydrogen (15 psi) in a Parr hydrogenation apparatus for 3.5 hr. The catalyst was filtered and the filtrate was concentrated to dryness in vacuo. The solid residue was chromatographed on silica gel (650g), eluting with 9:1 hexane/ethyl acetate to yield 14.0g. (85% yield) of the title compound as an off-white solid.

NMR ($CDCl_3$): δ 2.42 (s, 3H); 2.44 (s, 3H); 2.59 (s, 3H); 4.02 (b, 2H); 6.72 (s, 1H).

The title compounds of Examples 68-70 were prepared according to the procedure or Example 67.

## Claims

**Claims for the following Contracting States : AT, BE, CH, DE, DK, FR, GB, IT, LI, LU, NL, SE**

1. A compound of the formula

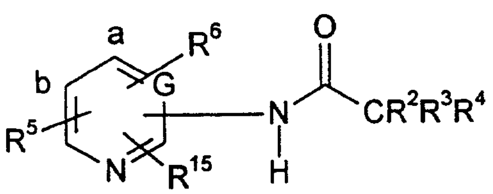

(I)

wherein $R^2$, $R^3$ and $R^4$ are

(a) independently selected from the group consisting of hydrogen, ($C_1$ - $C_4$) alkyl, A and $XR^{10}$; with the proviso that at least one of $R^2$, $R^3$ and $R^4$ must be A; or
(b) $R^2$ and $R^3$ together with the carbon atom to which they are attached form a cyclic or bicyclic system selected from the group consisting of ($C_3$-$C_7$) cycloalkyl, ($C_3$-$C_7$) cycloalkenyl, ($C_6$-$C_{14}$) bicycloalkyl, ($C_6$-$C_{14}$) bicycloalkenyl, and aryl-fused systems containing 8 to 15 carbon atoms, one ring of any of said aryl-fused systems being aromatic and the ring containing the carbon atom to which $R^2$ and $R^3$ are attached being non-aromatic; one of the carbon atoms of said aromatic ring being optionally replaced by sulfur or oxygen; one or more carbon atoms of said non-aromatic ring being optionally replaced by sulfur or oxygen, and one or more carbons of said aromatic ring being optionally replaced by nitrogen; one or two carbon atoms of said cycloalkyl or bicycloalkyl groups being optionally replaced by sulfur or oxygen; and said cyclic or bicyclic system being optionally substituted with one to five substituents independently selected from the group consisting of phenyl, substituted phenyl, $(C_1-C_6)$ alkyl and A, with the proviso that one and only one of said substituents is A, and one and only one of said substituents is phenyl or substituted phenyl, said substituted phenyl being substituted with one or more substituents independently selected from the group consisting of $(C_1-C_6)$ alkyl, $(C_1-C_6)$ alkylthio, halogen and trifluoromethyl; and $R^4$ is hydrogen, $XR^{10}$ or A;

A is a hydrocarbon containing 4 to 16 carbon atoms and 0, 1 or 2 double bonds:
X is S;
$R^5$ is $(C_1-C_6)$alkylthio;
$R^6$ is $(C_1-C_6)$alkylthio;
$R^{15}$ is $(C_1-C_4)$alkyl or $(C_1-C_6)$alkylthio;
$R^{10}$ is selected from the group consisting of $(C_4-C_{12})$ cycloalkyl, $(C_4-C_{12})$ straight or branched alkyl, $(C_4-C_{12})$ cycloalkyl-$(C_1-C_6)$ alkyl, phenyl-$(C_1-C_6)$ alkyl, (substituted phenyl)-$(C_1-C_6)$ alkyl, $(C_1-C_6)$ alkyl-phenyl, $(C_1-C_6)$ alkyl-(substituted phenyl) ; wherein the substituents on the substituted phenyl are selected from the group consisting of $(C_1-C_4)$ alkoxy, $(C_1-C_4)$ alkylthio, $(C_1-C_6)$ alkyl, halo and trifluoromethyl:
G is selected from the group consisting of nitrogen and carbon atoms with the proviso that when G is nitrogen, the resulting pyrimidine group is attached at the 4 or 5 position designated by a and b in formula 1 above;
or a pharmaceutically acceptable salt of said compound.

2. A compound according to claim 1 wherein G is N and the resulting group is 4,6-bis(methylthio)-2-methylpyrimidin-5-yl or G is CH and the resulting group is 2,4-bis(methylthio)-6-methylpyridin-3-yl.

3. A compound according to claim 1, said compound being selected from the group consisting of:

(2S)-N-[2,4-bis(methylthio)-6-methylpyridin-3-yl]-2-hexylthiodecanoic amide;
(2S)-N-[2-methyl-4,6-bis(methylthio)pyrimidin-5-yl]-2-hexylthiodecanoic amide;
N-[4,6-bis(methylthio)-2-methylpyrimidin-5-yl]-2-hexylthiodecanoic amide;
N-[2,4-bis(methylthio)-6-methylpyridin-3-yl]-2-hexylthiodecanoic amide;
N-[4,6-bis(methylthio)-2-methylpyrimidin-5-yl]-2,2-dimethyldocecanoic amide;
N-[2,4-bis(methylthio)-6-methylpyridin-3-yl]-2,2-dimethyldodecanoic amide;
N-[4,6-bis(methylthio)-2-methylpyrimidin-5-yl]-4,5-dimethyl-trans-2-heptylcyclohex-4-ene-carboxamide;
N-[4,6-bis(methylthio)-2-methylpyrimidin-5-yl]-2-heptylnonanoic amide;
N-[4,6-bis(methylthio)-2-methylpyrimidin-5-yl]pentadecanoic amide;
N-[2,4-bis(methylthio)-6-methylpyridin-3-yl-]pentadecanoic amide;
N-[2,4-bis(methylthio)-6-methylpyridin-3-yl]-(Z)-9-octadecenoic amide;
N-[4,6-bis(methylthio)-2-methylpyrimidin-5-yl]-(Z)-9-octadecenoic amide; and
N-[4,6-bis(methylthio)-2-methylpyrimidin-5-yl]-trans-3-nonyl-1,2,3,4-tetrahydro-2-naphthoic amide;

4. A pharmaceutical composition comprising a compound of the formula I as claimed in any of claims 1 to 3 together with a pharmaceutically acceptable carrier.

5. The use of a compound of the formula I as claimed in any one of claims 1 to 3 for preparation of a pharmaceutical composition for inhibiting acyl coenzyme A: cholesterol acyltransferase, inhibiting intestinal absorption of cholesterol, reversing or slowing the development of atherosclerosis, or lowering the concentration of serum cholesterol in a mammal.

**Claims for the following Contracting States : ES, GR**

1. A process for making a compound of the formula

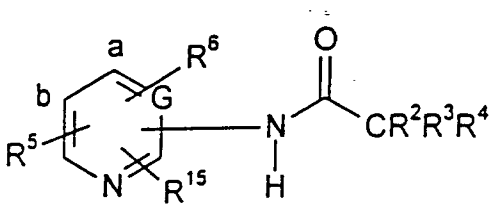

(I)

wherein $R^2$, $R^3$ and $R^4$ are

(a) independently selected from the group consisting of hydrogen, ($C_1$-$C_4$) alkyl, A and $XR^{10}$; with the proviso that at least one of $R^2$, $R^3$ and $R^4$ must be A; or

(b) $R^2$ and $R^3$ together with the carbon atom to which they are attached form a cyclic or bicyclic system selected from the group consisting of ($C_3$-$C_7$) cycloalkyl, ($C_3$-$C_7$) cycloalkenyl, ($C_6$-$C_{14}$) bicycloalkyl, ($C_6$-$C_{14}$) bicycloalkenyl, and aryl-fused systems containing 8 to 15 carbon atoms, one ring of any of said aryl-fused systems being aromatic and the ring containing the carbon atom to which $R^2$ and $R^3$ are attached being non-aromatic one of the carbon atoms of said aromatic ring being optionally replaced by sulfur or oxygen; one or more carbon atoms of said non-aromatic ring being optionally replaced by sulfur or oxygen, and one or more carbons of said aromatic ring being optionally replaced by nitrogen; one or two carbon atoms of said cycloalkyl or bicycloalkyl groups being optionally replaced by sulfur or oxygen; and said cyclic or bicyclic system being optionally substituted with one to five substituents independently selected from the group consisting of phenyl, substituted phenyl, ($C_1$-$C_6$) alkyl and A, with the proviso that one and only one of said substituents is A, and one and only one of said substituents is phenyl or substituted phenyl, said substituted phenyl being substituted with one or more substituents independently selected from the group consisting of ($C_1$-$C_6$) alkyl, ($C_1$-$C_6$) alkylthio, halogen and trifluoromethyl; and $R^4$ is hydrogen, $XR^{10}$ or A;

A is a hydrocarbon containing 4 to 16 carbon atoms and 0, 1 or 2 double bonds:
X is S;
$R^5$ is ($C_1$-$C_6$)alkylthio;
$R^6$ is ($C_1$-$C_6$) alkylthio;
$R^{15}$ is ($C_1$-$C_4$)alkyl or ($C_1$-$C_6$)alkylthio;
$R^{10}$ is selected from the group consisting of ($C_4$-$C_{12}$) cycloalkyl, ($C_4$-$C_{12}$) straight or branched alkyl, ($C_4$-$C_{12}$) cycloalkyl-($C_1$-$C_6$) alkyl, phenyl-($C_1$-$C_6$)alkyl,(subtituted phenyl)-($C_1$-$C_6$) alkyl, ($C_1$-$C_6$) alkyl-phenyl, ($C_1$-$C_6$) alkyl-(substituted phenyl); wherein the substituents on the substituted phenyl, are selected from the group consisting of ($C_1$-$C_4$) alkoxy, ($C_1$-$C_4$) alkylthio, ($C_1$-$C_6$) alkyl, halo and trifluoromethyl:
G is selected from the group consisting of nitrogen and carbon atoms with the proviso that when G is nitrogen, the resulting pyrimidine group is attached at the 4 or 5 position designated by a and b in formula 1 above;
or a pharmaceutically acceptable salt of said compound, comprising reacting a compound of the formula

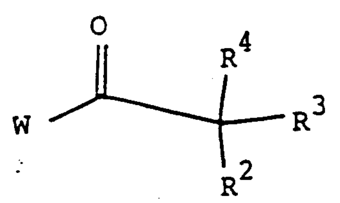

IV

wherein W is chloro or bromo and $R^2$, $R^3$ and $R^4$ are defined as above, with an amine of the formula $R^1NH_2$, wherein $R^1$ is a group of the formula

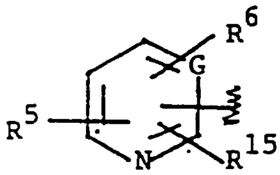

and an acid scavenger.

2. A process according to claim 1, wherein said compound of the formula IV is obtained by reacting a compound of the formula

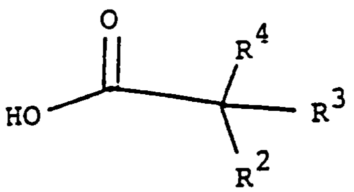

III

wherein $R^2$, $R^3$ and $R^4$ are defined as in claim 1, with a chlorinating or brominating agent.

3. A process according to claim 1 and claim 2, wherein said acid scavenger is selected from the group consisting of dimethylaminopyridine, pyridine and triethylamine, and said chlorinating or brominating agent is selected from the group consisting of oxalyl chloride, oxalyl bromide, thionyl chloride, thionyl bromide, phosphorus trichloride, phosphorous tribromide, phosphorous pentachloride, phosphorous pentabromide, phosphorous oxychloride and phosphorous oxybromide.

**Patentansprüche**

**Patentansprüche für folgende Vertragsstaaten : AT, BE, CH, DE, DK, FR, GB, IT, LI, LU, NL, SE**

1. Verbindung der Formel

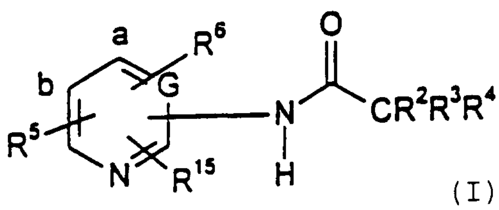

(I)

worin $R^2$, $R^3$ und $R^4$

(a) unabhängig ausgewählt sind aus der Gruppe, bestehend aus Wasserstoff, $(C_1$-$C_4)$-Alkyl, A und $XR^{10}$, mit der Maßgabe, daß mindestens einer der Reste $R^2$, $R^3$ und $R^4$ A sein muß, oder
(b) $R^2$ und $R^3$ zusammen mit dem Kohlenstoffatom, an das sie gebunden sind, ein cyclisches oder bicyclisches System bilden, ausgewählt aus der Gruppe, bestehend aus $(C_3$-$C_7)$-Cycloalkyl, $(C_3$-$C_7)$-Cycloalkenyl, $(C_6$-$C_{14})$-Bicycloalkyl, $(C_6$-$C_{14})$-Bicycloalkenyl und Aryl-kondensierten Systemen, die 8 bis 15 Kohlenstoffatome enthalten, wobei ein Ring der Arylkondensierten Systeme aromatisch ist und der Ring, der das Kohlenstoffatom enthält, an das $R^2$ und $R^3$ gebunden sind, nichtaromatisch ist; eines der Kohlenstoffatome des aromatischen Rings gegebenenfalls durch Schwefel oder Sauerstoff ersetzt ist; ein oder mehrere Kohlenstoffatome des nichtaromatischen Rings gegebenenfalls durch Schwefel oder Sauerstoff ersetzt sind, und ein oder mehrere Kohlenstoffatome des aromatischen Rings gegebenenfalls durch Stickstoff ersetzt sind; ein oder zwei Kohlenstoffatome der Cycloalkyl- oder Bicycloalkylgruppen gegebenenfalls durch Schwefel oder Sauerstoff

ersetzt sind, und das cyclische oder bicyclische System gegebenenfalls mit ein bis fünf Substituenten, unabhängig ausgewählt aus der Gruppe, bestehend aus Phenyl, substituiertem Phenyl, ($C_1$-$C_6$)-Alkyl und A, substituiert sind, mit der Maßgabe, daß einer und nur einer der Substituenten A ist, und einer und nur einer der Substituenten Phenyl oder substituiertes Phenyl ist, wobei das substituierte Phenyl mit ein oder mehreren Substituenten, unabhängig ausgewählt aus der Gruppe, bestehend aus ($C_1$-$C_6$)-Alkyl, ($C_1$-$C_6$)-Alkylthio, Halogen und Trifluormethyl, substituiert ist und $R^4$ Wasserstoff, $XR^{10}$ oder A darstellt;

A einen Kohlenwasserstoff, der 4 bis 16 Kohlenstoffatome und 0, 1 oder 2 Doppelbindungen enthält, darstellt:
X S darstellt,
$R^5$ ($C_1$-$C_6$)-Alkylthio darstellt,
$R^6$ ($C_1$-$C_6$)-Alkylthio darstellt,
$R^{15}$ ($C_1$-$C_4$)-Alkyl oder ($C_1$-$C_6$)-Alkylthio darstellt,
$R^{10}$ ausgewählt ist aus der Gruppe, bestehend aus ($C_4$-$C_{12}$)-Cycloalkyl, geradkettigem oder verzweigtem ($C_4$-$C_{12}$)-Alkyl, ($C_4$-$C_{12}$)-Cycloalkyl-($C_1$-$C_6$)-alkyl, Phenyl-($C_1$-$C_6$)-alkyl, (substituiertem Phenyl)-($C_1$-$C_6$)-alkyl, ($C_1$-$C_6$)-Alkylphenyl, ($C_1$-$C_6$)-Alkyl-(substituiertem Phenyl), darstellt, wobei die Substituenten an dem substituierten Phenyl ausgewählt sind aus der Gruppe, bestehend aus ($C_1$-$C_4$)-Alkoxy, ($C_1$-$C_4$)-Alkylthio, ($C_1$-$C_6$)-Alkyl, Halogen und Trifluormethyl;
G ausgewählt ist aus der Gruppe, bestehend aus Stickstoff- und Kohlenstoffatomen, mit der Maßgabe, daß, wenn G Stickstoff ist, die erhaltene Pyrimidingruppe an der 4- oder 5-Stellung, die in vorstehender Formel I mit a und b benannt ist, gebunden ist,
oder ein pharmazeutisch verträgliches Salz der Verbindung.

**2.** Verbindung nach Anspruch 1, wobei G N darstellt und die erhaltene Gruppe 4,6-Bis(methylthio)-2-methylpyrimidin-5-yl darstellt, oder G CH darstellt, und die erhaltene Gruppe 2,4-Bis(methylthio)-6-methylpyridin-3-yl darstellt.

**3.** Verbindung nach Anspruch 1, wobei die Verbindung ausgewählt ist aus der Gruppe, bestehend aus:

(2S)-N-[2,4-Bis(methylthio)-6-methylpyridin-3-yl]-2-hexylthiodecansäureamid,
(2S)-N-[2-Methyl-4,6-bis(methylthio)pyrimidin-5-yl]-2-hexylthiodecansäureamid,
N-[4,6-Bis(methylthio)-2-methylpyrimidin-5-yl]-2-hexylthiodecansäureamid,
N-[2,4-Bis(methylthio)-6-methylpyridin-3-yl]-2-hexylthiodecansäureamid,
N-[4,6-Bis(methylthio)-2-methylpyrimidin-5-yl]-2,2-dimethyldodecansäureamid,
N-[2,4-Bis(methylthio)-6-methylpyridin-3-yl]-2,2-dimethyldodecansäureamid,
N-[4,6-Bis(methylthio)-2-methylpyrimidin-5-yl]-4,5-dimethyl-trans-2-heptylcyclohex-4-en-carboxamid,
N-[4,6-Bis(methylthio)-2-methylpyrimidin-5-yl]-2-heptylnonansäureamid,
N-[4,6-Bis(methylthio)-2-methylpyrimidin-5-yl]pentadecansäureamid,
N-[2,4-Bis(methylthio)-6-methylpyridin-3-yl]pentadecansäureamid,
N-[2,4-Bis(methylthio)-6-methylpyridin-3-yl]-(Z)-9-octadecensäureamid,
N-[4,6-Bis(methylthio)-2-methylpyrimidin-5-yl]-(Z)-9-octadecensäureamid und
N-[4,6-Bis(methylthio)-2-methylpyrimidin-5-yl]-trans-3-nonyl-1,2,3,4-tetrahydro-2-naphthoesäureamid.

**4.** Pharmazeutische Zusammensetzung, umfassend eine Verbindung der Formel I nach einem der Ansprüche 1 bis 3, zusammen mit einem pharmazeutisch verträglichen Träger.

**5.** Verwendung einer Verbindung der Formel I nach einem der Ansprüche 1 bis 3, zur Herstellung einer pharmazeutischen Zusammensetzung zur Inhibierung von Acyl-Coenzym A: Cholesterinacyltransferase, Inhibierung der intestinalen Absorption von Cholesterin, Umkehrung oder Verlangsamung der Entwicklung von Arteriosklerose oder Senkung der Konzentration von Serumcholesterin bei einem Säuger.

**Patentansprüche für folgende Vertragsstaaten : ES, GR**

**1.** Verfahren zur Herstellung einer Verbindung der Formel

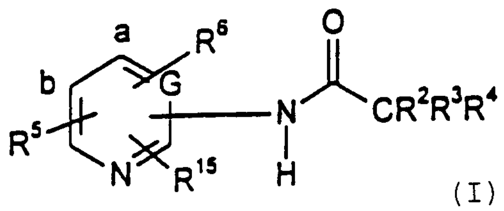

(I)

worin $R^2$, $R^3$ und $R^4$

(a) unabhängig ausgewählt sind aus der Gruppe, bestehend aus Wasserstoff, $(C_1-C_4)$-Alkyl, A und $XR^{10}$, mit der Maßgabe, daß mindestens einer der Reste $R^2$, $R^3$ und $R^4$ A sein muß, oder
(b) $R^2$ und $R^3$ zusammen mit dem Kohlenstoffatom, an das sie gebunden sind, ein cyclisches oder bicyclisches System bilden, ausgewählt aus der Gruppe, bestehend aus $(C_3-C_7)$-Cycloalkyl, $(C_3-C_7)$-Cycloalkenyl, $(C_6-C_{14})$-Bicycloalkyl, $(C_6-C_{14})$-Bicycloalkenyl und Aryl-kondensierten Systemen, die 8 bis 15 Kohlenstoffatome enthalten, wobei ein Ring der Arylkondensierten Systeme aromatisch ist und der Ring, der das Kohlenstoffatom enthält, an das $R^2$ und $R^3$ gebunden sind, nichtaromatisch ist; eines der Kohlenstoffatome des aromatischen Rings gegebenenfalls durch Schwefel oder Sauerstoff ersetzt ist; ein oder mehrere Kohlenstoffatome des nichtaromatischen Rings gegebenenfalls durch Schwefel oder Sauerstoff ersetzt sind, und ein oder mehrere Kohlenstoffatome des aromatischen Rings gegebenenfalls durch Stickstoff ersetzt sind; ein oder zwei Kohlenstoffatome der Cycloalkyl- oder Bicycloalkylgruppen gegebenenfalls durch Schwefel oder Sauerstoff ersetzt sind, und das cyclische oder bicyclische System gegebenenfalls mit ein bis fünf Substituenten, unabhängig ausgewählt aus der Gruppe, bestehend aus Phenyl, substituiertem Phenyl, $(C_1-C_6)$-Alkyl und A, substituiert sind, mit der Maßgabe, daß einer und nur einer der Substituenten A ist, und einer und nur einer der Substituenten Phenyl oder substituiertes Phenyl ist, wobei das substituierte Phenyl mit ein oder mehreren Substituenten, unabhängig ausgewählt aus der Gruppe, bestehend aus $(C_1-C_6)$-Alkyl, $(C_1-C_6)$-Alkylthio, Halogen und Trifluormethyl, substituiert ist und $R^4$ Wasserstoff, $XR^{10}$ oder A darstellt;

A einen Kohlenwasserstoff, der 4 bis 16 Kohlenstoffatome und 0, 1 oder 2 Doppelbindungen enthält, darstellt:
X S darstellt,
$R^5$ $(C_1-C_6)$-Alkylthio darstellt,
$R^6$ $(C_1-C_6)$-Alkylthio darstellt,
$R^{15}$ $(C_1-C_4)$-Alkyl oder $(C_1-C_6)$-Alkylthio darstellt,
$R^{10}$ ausgewählt ist aus der Gruppe, bestehend aus $(C_4-C_{12})$-Cycloalkyl, geradkettigem oder verzweigtem $(C_4-C_{12})$-Alkyl, $(C_4-C_{12})$-Cycloalkyl-$(C_1-C_6)$-alkyl, Phenyl-$(C_1-C_6)$-alkyl, (substituiertem Phenyl)-$(C_1-C_6)$-alkyl, $(C_1-C_6)$-Alkylphenyl, $(C_1-C_6)$-Alkyl-(substituiertem Phenyl), darstellt, wobei die Substituenten an dem substituierten Phenyl ausgewählt sind aus der Gruppe, bestehend aus $(C_1-C_4)$-Alkoxy, $(C_1-C_4)$-Alkylthio, $(C_1-C_6)$-Alkyl, Halogen und Trifluormethyl;
G ausgewählt ist aus der Gruppe bestehend aus Stickstoff- und Kohlenstoffatomen, mit der Maßgabe, daß, wenn G Stickstoff ist, die erhaltene Pyrimidingruppe an der 4- oder 5-Stellung, die in vorstehender Formel I mit a und b benannt ist, gebunden ist,
oder eines pharmazeutisch verträglichen Salzes der Verbindung, umfassend Umsetzen einer Verbindung der Formel

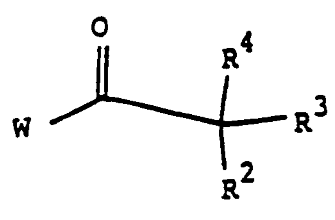

IV

worin W Chlor oder Brom darstellt und $R^2$, $R^3$ und $R^4$ wie vorstehend definiert sind, mit einem Amin der Formel $R^1NH_2$, worin $R^1$ eine Gruppe der Formel

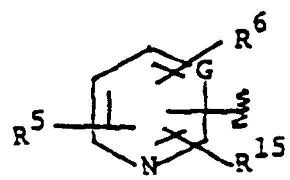

darstellt und einem Säurefänger.

2. Verfahren nach Anspruch 1, wobei die Verbindung der Formel IV durch Umsetzen einer Verbindung der Formel

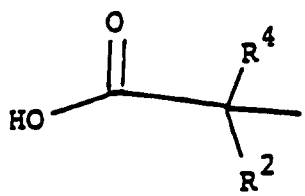

III

worin $R^2$, $R^3$ und $R^4$ wie in Anspruch 1 definiert sind, mit einem Chlorierungs- oder Bromierungsmittel, erhalten wird.

3. Verfahren nach Anspruch 1 oder Anspruch 2, wobei der Säurefänger ausgewählt ist aus der Gruppe, bestehend aus Dimethylaminopyridin, Pyridin und Triethylamin und das Chlorierungs-oder Bromierungsmittel ausgewählt ist aus der Gruppe, bestehend aus Oxalylchlorid, Oxalylbromid, Thionylchlorid, Thionylbromid, Phosphortrichlorid, Phosphortribromid, Phosphorpentachlorid, Phosphorpentabromid, Phosphoroxychlorid und Phosphoroxybromid.

**Revendications**

**Revendications pour les Etats contractants suivants : AT, BE, CH, DE, DK, FR, GB, IT, LI, LU, NL, SE**

1. Composé de formule

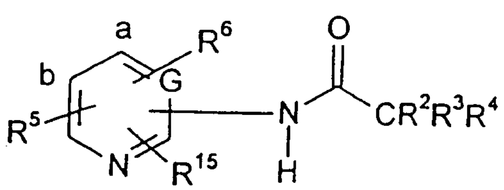

(I)

dans laquelle $R^2$, $R^3$ et $R^4$

(a) sont choisis indépendamment dans le groupe consistant en l'hydrogène, un reste alkyle en $C_1$ à $C_4$, A et $XR^{10}$, sous réserve que l'un au moins de $R^2$, $R^3$ et $R^4$ représente A ; ou bien
(b) $R^2$ et $R^3$ forment conjointement avec l'atome de carbone auquel ils sont liés un système cyclique ou bicyclique choisi dans le groupe consistant en un système cycloalkyle en $C_3$ à $C_7$, cycloalcényle en $C_3$ à $C_7$, bicycloalkyle en $C_6$ à $C_{14}$, bicycloalcényle en $C_6$ à $C_{14}$ et des systèmes condensés à un aryle et contenant 8 à 15 atomes de carbone, l'un des noyaux de l'un quelconque de ces systèmes condensés à un aryle étant aromatique et le noyau contenant l'atome de carbone auquel $R^2$ et $R^3$ sont liés étant non aromatique ; l'un des atomes de carbone de ce noyau aromatique étant facultativement remplacé par du soufre ou de l'oxygène ; un ou plusieurs atomes de carbone du noyau non aromatique étant facultativement remplacés par du soufre ou de l'oxygène, et un ou plusieurs atomes de carbone du noyau aromatique étant facultativement remplacés par de l'azote ; un ou deux atomes de carbone des groupes cycloalkyle ou bicycloalkyle étant facultativement remplacés par du soufre ou de l'oxygène ; et le système cyclique ou bicyclique étant facultativement substitué avec un à cinq substituants choisis indépendamment dans le groupe des substituants phényle ; phényle substitué,

alkyle en $C_1$ à $C_6$ et A, sous réserve qu'un et un seul de ces substituants représente A, et qu'un et un seul de ces substituants représente un groupe phényle ou phényle substitué, ce groupe phényle substitué portant un ou plusieurs substituants choisis indépendamment dans le groupe des substituants alkyle en $C_1$ à $C_6$, alkylthio en $C_1$ à $C_6$, halogéno et trifluorométhyle ; et $R^4$ est de l'hydrogène, un groupe $XR^{10}$ ou A ;

A est un hydrocarbure contenant 4 à 16 atomes de carbone et 0, 1 ou 2 doubles liaisons ;
X représente S ;
$R^5$ est un groupe alkylthio en $C_1$ à $C_6$ ;
$R^6$ est un groupe alkylthio en $C_1$ à $C_6$ ;
$R^{15}$ est un groupe alkyle en $C_1$ à $C_4$ ou alkylthio en $C_1$ à $C_6$ ;
$R^{10}$ est choisi dans le groupe consistant en un reste cycloalkyle en $C_4$ à $C_{12}$, alkyle linéaire ou ramifié en $C_4$ à $C_{12}$, (cycloalkyle en $C_4$ à $C_{12}$)-(alkyle en $C_1$ à $C_6$), phényl-(alkyle en $C_1$ à $C_6$), (phényle substitué)-(alkyle en $C_1$ à $C_6$), (alkyle en $C_1$ à $C_6$)-phényle, (alkyle en $C_1$ à $C_6$)-(phényle substitué), les substituants portés par le groupe phényle substitué étant choisis dans le groupe des substituants alkoxy en $C_1$ à $C_4$, alkylthio en $C_1$ à $C_4$, alkyle en $C_1$ à $C_6$, halogéno et trifluorométhyle ;
G est choisi dans le groupe consistant en des atomes d'azote et de carbone sous réserve que lorsque G est de l'azote, le groupe pyrimidine résultant soit lié dans la position 4 ou 5 désignée par a et b dans la formule 1 ci-dessus ;
ou un sel pharmaceutiquement acceptable de ce composé.

2. Composé suivant la revendication 1, dans lequel G représente N et le groupe résultant est le groupe 4,6-bis(méthylthio)-2-méthylpyrimidine-5-yle ou bien G représente CH et le groupe résultant est un groupe 2,4-bis(méthylthio)-6-méthylpyridine-3-yle.

3. Composé suivant la revendication 1, qui est choisi dans le groupe consistant en :

l'amide (2S)-N-[2,4-bis(méthylthio)-6-méthylpyridine-3-yl]-2-hexylthiodécanoïque ;
l'amide (2S)-N-[2-méthyl-4,6-bis(méthylthio)pyrimidine-5-yl]-2-hexylthiodécanoïque ;
l'amide N-[4,6-bis(méthylthio)-2-méthylpyrimidine-5-yl]-2-hexylthiodécanoïque ;
l'amide N-[2,4-bis(méthylthio)-6-méthylpyridine-3-yl]-2-hexylthiodécanoïque ;
l'amide N-[4,6-bis(méthylthio)-2-méthylpyrimidine-5-yl]-2,2-diméthyldodécanoïque ;
l'amide N-[2,4-bis(méthylthio)-6-méthylpyridine-3-yl]-2,2-diméthyldodécanoïque ;
le N-[4,6-bis(méthylthio)-2-méthylpyrimidine-5-yl]-4,5-diméthyl-trans-2-heptylcyclohex-4-ène-carboxamide ;
l'amide N-[4,6-bis(méthylthio)-2-méthylpyrimidine-5-yl]-2-heptylnonanoïque ;
l'amide N-[4,6-bis(méthylthio)-2-méthylpyrimidine-5-yl]pentadécanoïque ;
l'amide N-[2,4-bis(méthylthio)-6-méthylpyridine-3-yl]pentadécanoïque ;
l'amide N-[2,4-bis(méthylthio)-6-méthylpyridine-3-yl]-(Z)-9-octadécénoïque ;
l'amide N-[4,6-bis(méthylthio)-2-méthylpyrimidine-5-yl]-(Z)-9-octadécénoïque ; et
l'amide N-[4,6-bis(méthylthio)-2-méthylpyrimidine-5-yl] trans-3-nonyl-1,2,3,4-tétrahydro-2-naphtoïque.

4. Composition pharmaceutique comprenant un composé de formule I suivant l'une quelconque des revendications 1 à 3 en association avec un support acceptable du point de vue pharmaceutique.

5. Utilisation d'un composé de formule I suivant l'une quelconque des revendications 1 à 3 pour la préparation d'une composition pharmaceutique destinée à inhiber l'acylcoenzyme A : cholestérol-acyltransférase, à inhiber l'absorption intestinale du cholestérol, à faire rétrocéder ou à ralentir le développement de l'athérosclérose ou à abaisser la concentration en cholestérol du sérum chez un mammifère.

**Revendications pour les Etats contractants suivants : ES, GR**

1. Procédé de production d'un composé de formule

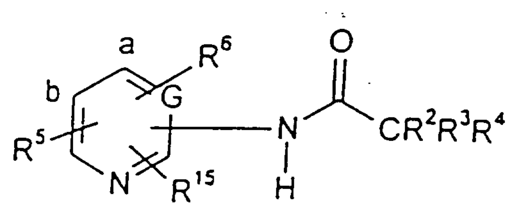

(I)

dans laquelle $R^2$, $R^3$ et $R^4$

(a) sont choisis indépendamment dans le groupe consistant en l'hydrogène, un reste alkyle en $C_1$ à $C_4$, A et $XR^{10}$, sous réserve que l'un au moins de $R^2$, $R^3$ et $R^4$ représente A ; ou bien

(b) $R^2$ et $R^3$ forment conjointement avec l'atome de carbone auquel ils sont liés un système cyclique ou bicyclique choisi dans le groupe consistant en un système cycloalkyle en $C_3$ à $C_7$, cycloalcényle en $C_3$ à $C_7$, bicycloalkyle en $C_6$ à $C_{14}$, bicycloalcényle en $C_6$ à $C_{14}$ et des systèmes condensés à un aryle et contenant 8 à 15 atomes de carbone, l'un des noyaux de l'un quelconque de ces systèmes condensés à un aryle étant aromatique et le noyau contenant l'atome de carbone auquel $R^2$ et $R^3$ sont liés étant non aromatique ; l'un des atomes de carbone de ce noyau aromatique étant facultativement remplacé par du soufre ou de l'oxygène ; un ou plusieurs atomes de carbone du noyau non aromatique étant facultativement remplacés par du soufre ou de l'oxygène, et un ou plusieurs atomes de carbone du noyau aromatique étant facultativement remplacés par de l'azote ; un ou deux atomes de carbone des groupes cycloalkyle ou bicycloalkyle étant facultativement remplacés par du soufre ou de l'oxygène ; et le système cyclique ou bicyclique étant facultativement substitué avec un à cinq substituants choisis indépendamment dans le groupe des substituants phényle, phényle substitué, alkyle en $C_1$ à $C_6$ et A, sous réserve qu'un et un seul de ces substituants représente A, et qu'un et un seul de ces substituants représente un groupe phényle ou phényle substitué, ce groupe phényle substitué portant un ou plusieurs substituants choisis indépendamment dans le groupe des substituants alkyle en $C_1$ à $C_6$, alkylthio en $C_1$ à $C_6$, halogéno et trifluorométhyle ; et $R^4$ est de l'hydrogène, un groupe $XR^{10}$ ou A ;

A est un hydrocarbure contenant 4 à 16 atomes de carbone et 0, 1 ou 2 doubles liaisons ;
X représente S ;
$R^5$ est un groupe alkylthio en $C_1$ à $C_6$ ;
$R^6$ est un groupe alkylthio en $C_1$ à $C_6$ ;
$R^{15}$ est un groupe alkyle en $C_1$ à $C_4$ ou alkylthio en $C_1$ à $C_6$ ;
$R^{10}$ est choisi dans le groupe consistant en un reste cycloalkyle en $C_4$ à $C_{12}$, alkyle linéaire ou ramifié en $C_4$ à $C_{12}$, (cycloalkyle en $C_4$ à $C_{12}$)-(alkyle en $C_1$ à $C_6$), phényl-(alkyle en $C_1$ à $C_6$), (phényle substitué)-(alkyle en $C_1$ à $C_6$), (alkyle en $C_1$ à $C_6$)-phényle, (alkyle en $C_1$ à $C_6$)-(phényle substitué), les substituants portés par le groupe phényle substitué étant choisis dans le groupe des substituants alkoxy en $C_1$ à $C_4$, alkylthio en $C_1$ à $C_4$, alkyle en $C_1$ à $C_6$, halogéno et trifluorométhyle ;
G est choisi dans le groupe consistant en des atomes d'azote et de carbone sous réserve que lorsque G est de l'azote, le groupe pyrimidine résultant soit lié dans la position 4 ou 5 désignée par a et b dans la formule 1 ci-dessus ;
ou d'un sel acceptable du point de vue pharmaceutique de ce composé,
comprenant la réaction d'un composé de formule

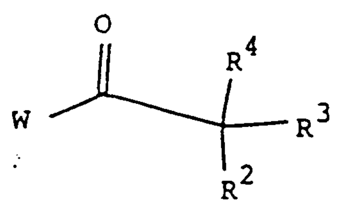

IV

dans laquelle W est un radical chloro ou bromo et $R^2$, $R^3$ et $R^4$ ont la définition donnée ci-dessus, avec une amine de formule $R^1NH_2$, dans laquelle $R^1$ est un groupe de formule

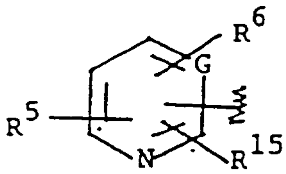

et d'un accepteur d'acide.

2. Procédé suivant la revendication 1, dans lequel le composé de formule IV est obtenu par réaction d'un composé de formule

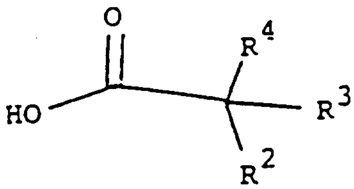

III

dans laquelle $R^2$, $R^3$ et $R^4$ sont tels que définis dans la revendication 1, avec un agent de chloration ou de bromation.

3. Procédé suivant la revendication 1 ou la revendication 2, dans lequel l'accepteur d'acide est choisi dans le groupe consistant en la diméthylaminopyridine, la pyridine et la triéthylamine et l'agent de chloration ou de bromation est choisi dans le groupe consistant en le chlorure d'oxalyle, le bromure d'oxalyle, le chlorure de thionyle, le bromure de thionyle, le trichlorure de phosphore, le tribromure de phosphore, le pentachlorure de phosphore, le pentabromure de phosphore, l'oxychlorure de phosphore et l'oxybromure de phosphore.